# EUROPEAN PATENT APPLICATION

(11) **EP 2 052 700 A1**
(43) Date of publication of application: **29.04.2009**
(21) Application number: 08450143.6
(22) Date of filing: 25.09.2008
(51) Int. Cl.: A61F 2/30

(54) **Drug delivery device and method for use with prosthetic device implantation**

(30) Priority: 26.09.2007 US 975504 P
(71) Applicant: Microchips, Inc., Bedford, MA 01730 (US)
(72) Inventor: Shelton, Kurt G., Woburn Massachusetts 01801 (US); James, Scott W., Danvers Massachusetts 01923 (US)
(74) Representative: Matschnig, Franz

(57) **Abstract**

Implantable drug delivery devices are provided which are adapted to be installed adjacent to at least a portion of an orthopedic prosthetic device implanted on a surgically prepared bone section of a patient. For example, the prosthetic device may be a hip joint replacement or knee joint replacement. The drug delivery device may include a body having a core formed from a non-biodegradable material formed in the shape of a sleeve and having an inner surface and an outwardly directed outer surface, wherein the outer surface has a drug formulation for release thereof into the body of the animal following implantation of the prosthetic device within the body of the animal. The outer surface may define one or more pockets for holding the drug formulation.

## Description

### Background of the Invention

This invention is generally in the field of implantable medical devices and methods for patient therapy and prophylaxis.

Implantable medical devices for the delivery of drugs to human or animal patients over extended periods are known. For example, U.S. Patent No. 5,797,898, U.S. Patent No. 6,527,762, U.S. Patent No. 6,491,666, and U.S. Patent No. 6,976,982, to Santini Jr. et al., describe devices for the storage and release of drug formulations from multi-reservoir devices.

It also known to have a bioabsorbable plug, containing a therapeutic agent, that is threaded to or inserted into a recess in a prosthesis, or is inserted into an implantable mesh bag that is sutured to soft tissue or a bone fastener, as in U.S. Patent No. 6,916,483 to Ralph et al.

It is also known to deliver a drug, such as an antibiotic, in conjunction with surgical implantation of various prosthetic devices, such as orthopedic or dental devices. For example, it is known to include an antibiotic in bone cement used to secure an orthopedic implant. *See* Jiranek, et al., "Antibiotic-Loaded Bone Cement for Infection Prophylaxis in Total Joint Replacement," J. Bone Joint Surg. Am. 88:2487-500 (2006). U.S. Patent No. 6,916,483 to Ralph et al.; U.S. Patent Application Publication No. 2007/0088442 to Cima et al.; U.S. Patent Application Publication No. 2007/0016163 to Santini Jr. et al.; and U.S. Patent Application Publication No. 2006/0093646 to Cima et al. describe devices for the release of a drug from an orthopedic or dental implant device. U.S. Patent Application Publication No. 2006/0093646 also discloses at paragraph [0068] a flexible drug delivery device, formed of a polymeric film containing pellets of a therapeutic agent, that is wrapped around bone tissue.

It would be desirable to provide improved or alternative techniques and devices for controlled local delivery of one or more therapeutic or prophylactic agents to the bodily tissues surrounding a site of implantation of a prosthetic implant device. These techniques and devices desirably would provide well-controlled delivery of the drug(s) over an extended period, such as several days, weeks, or months. The drug delivery devices desirably would be relatively simple to implant during the prosthetic implantation surgery, would not interfere with operation of the prosthetic device, and would not require explantation following completion of drug delivery.

### Summary of the Invention

In one aspect, implantable drug delivery devices are provided which are adapted to be installed adjacent to at least a portion of an orthopedic prosthetic device implanted on a surgically prepared bone section of a patient. The drug delivery device may include a body having a core formed from a non-biodegradable material formed in the shape of a sleeve and having an inner surface and an outwardly directed outer surface, wherein the outer surface has a drug formulation for release thereof into the body of the animal following implantation of the prosthetic device within the body of the patient. The body of the drug delivery device is shaped for association with at least a portion of one or both of the prosthetic device and the surgically prepared bone section. The outer surface may define at least one pocket for holding the drug formulation.

In one embodiment of the drug delivery device, the body has a radially inwardly directed inner surface, and a radially outwardly directed outer surface, and the inner surface defines an aperture that extends though the device body. The body may define an open shape having two free ends. The body of the drug delivery holds a drug formulation for *in vivo* release through the outer surface following implantation of the prosthetic device within the patient and installation of the drug delivery device.

In one particular embodiment, the prosthetic device is a hip replacement joint having a femoral portion including a hip stem having a neck, wherein the body of the drug delivery device is adapted to receive a portion of said hip stem in confronting relation around three sides of the hip stem or to receive the hip stem with the inner surface of the body in surrounding relation to the neck. In another particular embodiment, the prosthetic device is a knee replacement joint having an articular surface, wherein the body is adapted to engage a peripheral portion of the articular surface and the body in the shape of a sleeve has an approximate C-shaped extent, portions of the body being smooth in regions to be overlaid by muscle or tendon tissue.

In another aspect, a method is provided for delivering a drug to a human or other mammalian animal patient, in which an orthopedic prosthetic device is implanted. The method includes implanting the orthopedic prosthetic device onto a surgically prepared bone section of the human or other animal, and surrounding at least a portion of the prosthetic device with an implantable drug delivery device. Preferably, the combination is adapted to prevent relative motion between the prosthetic device and the drug delivery device.

### Brief Description of the Drawings

**FIG. 1** is a perspective view of an embodiment of a drug delivery device that is adapted for use with a standard knee prosthesis.
**FIG. 2** is a top view of the embodiment of the drug delivery device shown in FIG. **1**.
**FIG. 3** is a front view of the embodiment of the drug delivery device shown in **FIG. 1**.
**FIG. 4** is a back view of the embodiment of the drug delivery device shown in **FIG. 1****.**
**FIG. 5** is a left side view of the embodiment of the drug delivery device shown in **FIG. 1****.**
**FIG. 6** is a front perspective view of the embodiment of the drug delivery device shown in **FIG. 1****,** illustrating the device implanted adjacent to an implanted knee prosthesis, with a front portion of the device positioned behind the patellar tendon and a left portion of the device positioned behind the lateral collateral ligament.
**FIG. 7** is a front perspective view of the drug delivery device implanted as shown in **FIG. 6**, illustrating the front portion of the device adjacent to the articular surface of the knee prosthesis with the patella and the patellar tendon removed.
**FIG. 8** is a left rear perspective view of the drug delivery device implanted as shown in FIG. 6, illustrating the left portion of the device positioned behind the lateral collateral ligament.
**FIG. 9** is a right side view of the drug delivery device implanted as shown in **FIG. 6****,** illustrating the right portion of the device positioned beneath the medial collateral ligament.
**FIGS. 10** is another top view of the embodiment of the drug delivery device shown in **FIG. 1**.
FIG. 11 is a cross-sectional view of the embodiment of the drug delivery device taken along line C-C in **FIG. 10****.**
**FIG. 12** is a rear perspective view of the embodiment of the drug delivery device taken along line D-D in **FIG. 10****.**
**FIG. 13** is another side view of the embodiment of the drug delivery device shown in **FIG. 1****.**
**FIG. 14** is a cross-sectional view of the embodiment of the drug delivery device taken along line A-A in **FIG. 13****.**
**FIG. 15** is a cross-sectional view of the embodiment of the drug delivery device taken along line B-B in **FIG. 13****.**
**FIG. 16** is a perspective view of an embodiment of the drug delivery device shown in FIG. 1, illustrating example dimensions of the drug delivery device.
**FIG. 17** is a front view of the embodiment of the drug delivery device shown in **FIG. 16**, illustrating example dimensions of the drug delivery device.
**FIG. 18** is a rear view of the embodiment of the drug delivery device shown in **FIG. 16****,** illustrating example dimensions of the drug delivery device.
**FIG. 19** is a top view of the embodiment of the drug delivery device shown in **FIG. 16****,** illustrating example dimensions of the drug delivery device.
**FIG. 20** is a top view of the embodiment of the drug delivery device shown in **FIG. 16****,** illustrating example dimensions of the drug delivery device.
**FIG. 21** is a bottom view of the embodiment of the drug delivery device shown in **FIG. 16**, illustrating example dimensions of the drug delivery device.
**FIG. 22** is an upper perspective view of an embodiment of a drug delivery device adapted for use with a standard hip prosthesis.
**FIG. 23** is a lower perspective view of the embodiment of the drug delivery device shown in **FIG. 22****.**
**FIG. 24** is a perspective view of the embodiment of the drug delivery device shown in **FIG. 22****,** illustrating the device implanted adjacent to a portion of an implanted hip prosthesis.
**FIG. 25** is another perspective view of the embodiment of the drug delivery device shown in **FIG. 22****,** illustrating the device positioned about a portion of an implanted hip prosthesis with a head and an acetabular cup of the hip prosthesis removed so that a neck of the hip prosthesis is visible.
**FIG. 26** is a side view of the embodiment of the drug delivery device shown in **FIG. 22****,** illustrating the device implanted adjacent to a portion of an implanted hip prosthesis with a head and an acetabular cup of the hip prosthesis removed.
**FIG. 27** is a side view of the embodiment of the drug delivery device shown in **FIG. 22****,** illustrating the device implanted adjacent to a portion of an implanted hip prosthesis with a head and an acetabular cup of the hip prosthesis removed.
**FIG. 28** is a front view of the embodiment of the drug delivery device shown in **FIG. 22****,** illustrating the device implanted adjacent to a portion of an implanted hip prosthesis, with an acetabular cup in a non-rotated position.
**FIG. 29** is a another front view of the embodiment of the drug delivery device shown in **FIG. 22**, illustrating the device implanted adjacent to a portion of an implanted hip prosthesis, with an acetabular cup in a rotated position.
**FIG. 30** is a top view of the embodiment of the drug delivery device shown in **FIG. 22**, illustrating the device implanted adjacent to a portion of an implanted hip prosthesis with a head and an acetabular cup of the hip prosthesis removed.
**FIG. 31** is an upper perspective view of another embodiment of a drug delivery device adapted for use with a standard hip prosthesis.
**FIG. 32** is a lower perspective view of the embodiment of the drug delivery device shown in **FIG. 31****.**
**FIG. 33** is a perspective view of the embodiment of the drug delivery device shown in **FIG. 31****,** illustrating the device positioned about a portion of an implanted hip prosthesis with a head and an acetabular cup of the hip prosthesis removed so that a neck of the hip prosthesis is visible.
**FIG. 34** is a side view of the embodiment of the drug delivery device shown in **FIG. 31****,** illustrating the device implanted adjacent to a portion of an implanted hip prosthesis with a head and an acetabular cup of the hip prosthesis removed.
**FIG. 35** is a top view of the embodiment of the drug delivery device shown in **FIG. 31**, illustrating the device implanted adjacent to a portion of an implanted hip prosthesis with a head and an acetabular cup of the hip prosthesis removed.
**FIG. 36** is another perspective view of the embodiment of the drug delivery device shown in **FIG. 31****,** illustrating a set screw maintaining a position of the device against a hip stem of the implanted hip prosthesis.
**FIG. 37** is a side view of the embodiment of the drug delivery device shown in **FIG. 31****,** further illustrating the set screw maintaining the position of the device against the hip stem of the implanted hip prosthesis.
**FIGS. 38-41** are additional perspective, top, side, and bottom views of the embodiment of the drug delivery device shown in **FIG. 31**, illustrating example dimensions of the drug delivery device.
**FIG. 42** is a side view of the embodiment of the drug delivery device taken along perspective A-A in **FIG. 39****,** illustrating example dimensions of the drug delivery device.
**FIG. 43** is a cross-sectional view of the embodiment of the drug delivery device taken along line C-C in **FIG. 40****,** illustrating example dimensions of the drug delivery device.
**FIG. 44** is a cross-sectional view of the embodiment of the drug delivery device taken along line D-D in **FIG. 40****,** illustrating example dimensions of the drug delivery device.
**FIG. 45** is a view of the embodiment of the drug delivery device taken along line E-E in FIG. 44, illustrating example dimensions of the drug delivery device.
**FIG. 46** is a view of the embodiment of the drug delivery device taken along line K-K in **FIG. 45****,** illustrating example dimensions of the drug delivery device.
**FIG. 47** is a rear view of the embodiment of the drug delivery device shown in **FIG. 38**, illustrating example dimensions of the drug delivery device.
**FIG. 48** is a view of the embodiment of the drug delivery device taken along line H-H in **FIG. 47****,** illustrating example dimensions of the drug delivery device.
**FIG. 49** is a perspective view of an embodiment of a drug delivery device formed in the shape of a closed sleeve or cap.
**FIG. 50** is a perspective view of the embodiment of the drug delivery device shown in **FIG. 49**, illustrating the device positioned about an upper portion of a hip implant.
**FIG. 51** is a side view of the embodiment of the drug delivery device shown in FIG. 49, illustrating the device positioned about an upper portion of a hip implant.
**FIG. 52** is a top perspective view of the embodiment of the drug delivery device shown in **FIG. 49****,** illustrating the device positioned about an upper portion of a hip implant.
**FIG. 53** is a perspective cross-sectional view of the embodiment of the drug delivery device shown in **FIG. 49****,** illustrating protruding flat areas that assist in securing the device to the hip implant, and corresponding relief areas.
**FIG. 54** is a front cross-sectional view of the embodiment of the drug delivery device shown in **FIG. 49**, taken through the relief areas.
**FIG. 55** is a perspective cross-sectional view of the embodiment of the drug delivery device shown in **FIG. 49****,** taken through the protruding flat areas.
**FIG. 56** is a perspective cross-sectional view of another embodiment a drug delivery device, which is adapted to be secured to the hip implant with cement.
**FIG. 57** is a cross-sectional view of another embodiment of a drug delivery device, which is molded to mate with a tapered hip stem.
**FIG. 58** is a cross-sectional view of an embodiment of a tapered hip stem, which includes multiple discrete reservoirs for holding a drug formulation.

### Detailed Description of the Invention

Implantable drug delivery devices have been developed for use in combination with an implantable prosthetic device, such as in a partial or complete joint replacement. For example, the drug delivery device may be used with various hip or knee replacement surgeries, for example for infection prophylaxis. The devices advantageously provide controlled, local delivery of antibiotic agents and/or other drugs to the site of implantation of the prosthetic implant. For example, the device may serve as a universal drug delivery device that can easily be incorporated into total hip arthoplasty (THA) or a total knee arthroplasty (TKA) to provide precise local dosing and pharmacokinetics from formulations optimized for stability, while avoiding late elution.

The devices advantageously can provide controlled delivery of known quantities of antibiotics or other drugs, more precisely than from a cement, such as in a procedure where the prosthesis is secured using a drug-containing bone cement, the use of which may limit the selection or doses of drug that can be used without comprising cement strength. In addition, the present device can provide drug delivery with implant procedures that are cementless.

The devices beneficially can be used with various commercially-available or other prosthetic implant devices and generally do not require modification of the prosthetic device design in order to provide the drug delivery functionality. Advantageously, the devices may be directly secured primarily or partially to the implanted joint prosthetic device, and not exclusively secured to patient tissues at the site of implantation.

In a preferred embodiment, the devices release the drug from discrete reservoirs (also referred to as "pockets") defined in the body, rather than from a monolithic device body, which confers several advantages including decoupling the mechanical properties of the device from the drug formulation, easing delivery of multiple drugs (without concern of drug incompatibilities in a single formulation), and protecting/stabilizing of the drug formulation in the device.

### The Devices

In one aspect, an implantable drug delivery device is provided which is adapted to be installed at or adjacent an implantable orthopedic prosthetic device inserted onto a surgically prepared bone section of an animal, such as a human or other mammalian patient. In one embodiment, the implantable drug delivery device includes a body formed in the shape of a sleeve and having an inner surface and an outwardly directed outer surface, the outer surface having a therapeutic agent for release thereof into the body of the animal following implantation of the prosthetic device within the body of the animal.

In one embodiment, the sleeve defines an open shape having free ends. In another embodiment, the sleeve defines a closed annular shape. In various embodiments, the body of the device may be formed of a biocompatible metal, polymer, or ceramic. For example, the body may be made of an alloy of titanium such as titanium-aluminum-vanadium (such as Ti-6Al-4V), a stainless steel, a cobalt chrome alloy, zirconia, or a combination thereof. In another embodiment, the body may be formed of a non-degradable polymer, such as a cross-linked or non-cross-linked polyethylene, and is preferably rigid. In another embodiment, the body may be formed of a degradable polymer, such as poly(lactic-co-glycolic acid) (PLGA), poly(lactic acid)s, poly(glycolic acid)s, or degradable poly(anhydride-co-imides), a polytetrafluoroethylene, a polyester, a silicone, or a combination thereof. The body may be a composite or multilayer structure. The degradable polymer may be a drug-eluting polymeric material.

In one embodiment, the device body is formed of a drug-eluting polymer. The drug may be loaded in the polymer, such as homogeneously dispersed throughout a polymeric matrix material or heterogeneously distributed throughout the matrix material.

In one embodiment, the outer surface of the body defines one or more pockets, i.e., reservoirs, for holding at least one therapeutic agent. For example, an array of several discrete reservoirs may be provided in/on one or more surface regions of the implant device. The number of reservoirs and the size of the reservoirs may vary depending on, for example, the "real estate" available on or in the implant device for holding the reservoirs and/or the total volume of drug (or drug formulation) to be implanted in and delivered to the patient. For example, the total volume of the reservoirs in a single device may be between about 0.5 mL and about 5 mL, e.g., about 2 mL or about 3 mL. A reservoir may extend substantially into or through the body structure. The shape and dimensions of the reservoir, along with the number and size of the reservoir openings in the surface of the device body, can be selected to influence the rate of drug diffusion from a reservoir.

In various embodiments, the reservoirs are discrete, non-deformable, and disposed in an array in the body. The reservoir openings may be along one or more surface regions of the device body, and may be provided around surfaces to release drug in multiple directions. The body may include tens or hundreds of reservoirs arrayed across the exterior surface. The body may be substantially rigid and the reservoirs may be non-deformable.

In one embodiment, the reservoirs are macroreservoirs. A "macroreservoir" is a reservoir suitable for storing and releasing/exposing a quantity of material larger than a microquantity. The macroreservoir has a volume greater than 500 µL (e.g., greater than 600 µL, greater than 750 µL, greater than 900 µ, greater than 1 mL, etc.) and less than 10 mL (e.g., less than 5 mL, less than 3 mL, less than 2 mL, less than 1 mL, etc.). In one embodiment, the reservoirs are microreservoirs. A "microreservoir" is a reservoir suitable for storing and releasing/exposing a microquantity of material, such as a drug formulation. The term "microquantity" refers to volumes from 1 nL up to 500 µL. In one embodiment, the microreservoir has a volume equal to or less than 500 µL (e.g., less than 250 µL, less than 100 µL, less than 50 µ, less than 25 µL, less than 10 µL, etc.) and greater than about 1 nL (e.g., greater than 5 nL, greater than 10 nL, greater than about 25 nL, greater than about 50 nL, greater than about 1 µL, etc.). In one embodiment, the microquantity is between 1 nL and 1 µL. In another embodiment, the microquantity is between 10 nL and 500 nL. In still another embodiment, the microquantity is between about 1 µL and 500 µL. Unless explicitly indicated to be limited to either micro- or macro-scale volumes/quantities, the term "reservoir" is intended to encompass both.

In a preferred embodiment, the reservoirs and drug formulation are designed such that the formulation, which typically is in a solid form, cannot be released from the reservoir as a whole (e.g., as a pellet) but rather can only be released in a gradual, top down fashion as the drug formulation dissolves/diffuses. This may be controlled, for example, by the shape of the reservoir (e.g., a reservoir opening narrower than the distal periphery of the drug formulation pellet), the interior surface of the reservoir (e.g., a rough surface), the use of sticky or viscous excipient materials with the drug, the use of a flexible liner material lining the reservoir interior, and/or the use of a relatively flexible body material (e.g., silicone, polymer).

In an alternative embodiment, the device body, or a portion thereof such as the outer surface, is formed of a drug-eluting polymer and has no discrete drug-containing reservoirs located about the surface of the device, such that the drug is loaded in the polymer, e.g., homogeneously dispersed throughout a polymeric matrix material or heterogeneously distributed throughout the matrix material.

As used herein, the terms "drug" and "therapeutic agent" are used interchangeably. These terms include any therapeutic or prophylactic active pharmaceutical ingredient (API). A variety of different kinds of drugs may be released from the implant device. The drug may be one that is useful for pain management, infection control, osteointegration, thrombophrophylaxsis, oncology, or a combination thereof. In a preferred embodiment, the therapeutic agent is an antibiotic, such as gentamicin, vancomycin, erythromycin, tobramycin, cefazolin, ciprofloxacin, clindamycin, ticarcillin, cefuroxime, cefotaxime, fusidic acid, or a combination thereof. Other aminoglycoside or cephalosporin antibiotics may also be used in infection prophylaxis. In one example, the device is used to deliver one or more antibiotics for at least 30 days at therapeutically effective levels. In another example, the device is used to deliver one or more antibiotics for up to 3 months at therapeutically effective levels.

In one embodiment, multiple antibiotics may be delivered from different reservoirs for a synergistic effect, potentially at lower doses per antibiotic.

In one embodiment, the device includes a plurality of pockets, where a subset of the pockets has a different formulation than that of another subset of the pockets. For example, different pockets may have different drugs or different formulations of the same drug. The pockets may be tailored to provide different kinetics of release. This may be accomplished by providing the drug in the form of multiple discrete layers of drug and a non-bioactive, release controlling substance such as a biodegradable polymer, or by providing the drug in the form of a gradient composition, in order to provide staged, controlled release.

As used herein, the term "drug formulation" refers to a composition that comprises a drug. The drug formulation may include one or more pharmaceutically acceptable excipients, which are known in the art.

The drug formulation may be in essentially any form, such as a pure solid or liquid, a gel or hydrogel, a solution, an emulsion, a slurry, or a suspension. In a preferred embodiment, the drug formulation is in a monolithic, dry solid form, or in the form of a collection of particles (e.g., microparticles or nanoparticles), particularly for purposes of maintaining or extending the stability of the drug over a commercially and medically useful time, such as during storage in a drug delivery device until the drug needs to be administered. These solid forms may be provided by lyophilization of a drug solution or suspension directly in the reservoirs. Alternatively, prefabricated pellets that are approximately the size of the individual reservoirs may be formed outside the reservoirs (e.g., in a mold) and then may be transferred and deposited into the reservoirs, such as by a pick-and-place technique. Alternatively, the drug formulation may be in a gel, liquid, or suspension form. The particular formulation in the reservoirs of a single device may be the same as or different from one another across a plurality of the reservoirs.

The drug formulation may include a drug in combination with other materials to control or enhance the rate and/or time of release from an opened reservoir. In various embodiments, the drug formulation further includes one or more matrix materials. In one example, the matrix material comprises one or more synthetic polymers. Exemplary materials include synthetic polymers, such as PLGA, PEG, and poly-L-lactide (PLLA), and/or naturally occurring polymers such as hyaluronic acid, chitosan, and alginate. The naturally occurring polymers may or may not be crosslinked by methods known to the art. In another example, the one or more matrix materials may comprise a biodegradable, bioerodible, water-soluble, or water-swellable matrix material. In one embodiment, the drug is distributed in the matrix material and the matrix material degrades or dissolves in *vivo* to controllably release the therapeutic or prophylactic agent. The drug may be heterogeneously distributed in the reservoir or may be homogeneously distributed in the reservoir. The matrix material can be a "release system," as described in U.S. Patent No. 5,797,898, the degradation, dissolution, or diffusion properties of which can provide a method for controlling the release rate of the drug molecules.

Representative examples of other drugs that may be useful with the present devices include anti-thrombotic agents, anti-inflammatories, vaccines, vectors for gene therapy, polypeptides, nucleic acids (DNA, siRNA), interferons, antibodies, hormones, and chemotherapeutic agents. In various embodiments, the drug can be selected from amino acids, vaccines, antiviral agents, gene delivery vectors, interleukin inhibitors, immunomodulators, neurotropic factors, neuroprotective agents, antineoplastic agents, chemotherapeutic agents, polysaccharides, anti-coagulants (e.g., LMWH, pentasaccharides), antibiotics (e.g., immunosuppressants), analgesic agents, and vitamins. The drug may be a protein, such as glycoproteins, enzymes (e.g., proteolytic enzymes), hormones or other analogs (e.g., LHRH, steroids, corticosteroids, growth factors), antibodies (e.g., anti-VEGF antibodies, tumor necrosis factor inhibitors), cytokines (e.g., α-, β-, or y-interferons), interleukins (e.g., IL-2, IL-10), and diabetes/obesity-related therapeutics (e.g., insulin, exenatide, PYY, GLP-1 and its analogs). The drug may be a gonadotropin-releasing hormone (LHRH) analog, such as leuprolide. The drug may comprise a parathyroid hormone, such as a human parathyroid hormone or its analogs, e.g., hPTH(1-84) or hPTH(1-34). The drug may be selected from nucleosides, nucleotides, and analogs and conjugates thereof. The drug may comprise a peptide with natriuretic activity. The drug may be selected from diuretics, vasodilators, inotropic agents, anti-arrhythmic agents, Ca⁺ channel blocking agents, anti-adrenergics/sympatholytics, and renin angiotensin system antagonists. The drug may be a VEGF inhibitor, VEGF antibody, VEGF antibody fragment, or another anti-angiogenic agent. The drug may be a prostaglandin, a prostacyclin, or another drug effective in the treatment of peripheral vascular disease. The drug may be an angiogenic agent, such as VEGF. The drug may be an anti-inflammatory, such as dexamethasone. A single device may include a single drug or a combination of two or more drugs.

The release of drug from a single reservoir may be tailored to provide a temporally modulated release profile (e.g., pulsatile release), such as when time variation in plasma levels is desired, or a more continuous or consistent release profile, such as when constant plasma levels are needed to enhance a therapeutic effect, for example. Pulsatile release can be achieved from an individual reservoir, from a plurality of reservoirs, or a combination thereof. For example, where each reservoir provides only a single pulse, multiple pulses (i.e., pulsatile release) are achieved by temporally staggering the single pulse release from each of several reservoirs. Alternatively, multiple pulses can be achieved from a single reservoir by incorporating several layers of a release system and other materials into a single reservoir. Continuous release can be achieved by incorporating a release system that degrades, dissolves, or allows diffusion of molecules through it over an extended period. In addition, continuous release can be approximated by releasing several pulses of molecules in rapid succession ("digital" release). In one embodiment, the drug formulation within a reservoir comprises layers of a drug or drugs and a non-drug material, wherein the multiple layers provide pulsed drug release due to the intervening layers of non-drug. Such a strategy can be used to obtain complex release profiles.

In one embodiment, the body further includes a fixation means for securing the device to the prosthetic device, the bone tissue, or both. For example, the fixation means may be chosen from the group of fixation means consisting of: a hole adapted to receive a screw, a pin, a suture, a cement, or a glue; a detent adapted to engage a groove; an indentation adapted to receive a cement or glue; a snap fitting; a pressure fitting; a screw fitting; a screw; a pin; a suture; a cement; a glue; and a groove. Bone cements are well known in the art, and for example, may be a polymethylmethacrylate. The fixation means enables the surgeon to secure the device at the desired site once placed in adjacent to the surgically prepared bone section and/or the implant.

In one embodiment, the prosthetic device is a portion of a hip or knee replacement joint. In one particular example, the prosthetic device is a hip replacement joint having a femoral portion including a hip stem having a neck, wherein in the drug delivery device (i) a portion of the sleeve is adapted to be cemented to at least a portion of the hip stem or the surgically prepared bone section, and (ii) the aperture is adapted to receive a portion of the hip stem in confronting relation around three sides of the hip stem. In another example, the prosthetic device is a hip replacement joint having a femoral portion including a hip stem having a neck, wherein in the drug delivery device the sleeve is adapted to receive the hip stem with the inner surface in surrounding relation to the neck.

In yet another example, the prosthetic device is a knee replacement joint having an articular surface, and the drug delivery device the sleeve defines an open shape having free ends, wherein (i) the sleeve is adapted to engage a peripheral portion of the articular surface; and (ii) the sleeve has an approximate C-shaped extent, portions of the sleeve being smooth in regions to be overlaid by muscle, tendon, or ligament tissue.

In another aspect, an implantable drug delivery device is provided which is adapted to be installed at or adjacent an implantable orthopedic prosthetic device inserted onto a surgically prepared bone section of an animal. The device includes a body having a radially inwardly directed inner surface defining an aperture, and a radially outwardly directed outer surface; said body having a thickness dimension defined in a generally radial direction extending from said inner surface to said outer surface; wherein said aperture extends over the length of the thickness dimension; and wherein said outer surface holds a therapeutic agent for release thereof into the body of the animal following implantation of the prosthetic device within the body of the animal.

In one embodiment of this drug delivery device, the prosthetic device is a portion of a hip or knee replacement joint. The prosthetic device may be a hip replacement joint having a femoral portion including a hip stem having a neck, wherein (i) said body is adapted to be cemented to at least a portion of said hip stem or said surgically prepared bone section, and (ii) said aperture is adapted to receive a portion of said hip stem in confronting relation around three sides of said hip stem.

In another aspect, an implantable drug delivery device is provided which is adapted to be installed on an implantable orthopedic prosthetic device inserted onto a surgically prepared bone section of an animal, wherein the device includes a body having a first wall and an outer surface, said first wall adapted to be cemented to at least one of a portion of said implantable prosthetic device or said surgically prepared bone section; an aperture formed in said body and extending through said body, said aperture adapted to receive a portion of said implantable prosthetic device; and said outer surface holds a therapeutic agent for release thereof into the body of the animal following implantation of the prosthetic device within the body of the animal.

The outer surface may define at least one pocket for holding said therapeutic agent, which may be for example an antibiotic. The body may further include a fixation means chosen from the group of fixation means consisting of one or more holes, pins, detents, and indentations for receiving a cement.

The prosthetic device may be a portion of a hip or knee replacement joint. In one embodiment, the prosthetic device is a hip replacement joint having a femoral portion including a hip stem having a neck, wherein (i) said first wall is adapted to be cemented to at least a portion of said hip stem or said surgically prepared bone section, and (ii) said aperture is adapted to receive a portion of said hip stem in confronting relation around three sides of said hip stem.

Reservoir openings optionally may be closed off by at least one reservoir cap, membrane, film, or other structure. For example, each reservoir may further include a discrete reservoir cap. A reservoir cap may cover the opening(s) of the reservoir to protect the drug formulation until it is desired to initiate release or the reservoir cap may serve as a diffusion limiting, i.e., release rate controlling, material. A reservoir cap is a thin film or other structure suitable for separating the contents of a reservoir from the environment outside of the reservoir. The reservoir caps are formed from a material or mixture of materials that degrade, dissolve, or otherwise disintegrate in *vivo,* or that do not degrade dissolve, or disintegrate, but become permeable *in vivo* to the drug molecules.

The device body or reservoir cap may include a wrap or coating of a semi-permeable or degradable material, such as a polymer, to control transport of molecules into or out of the reservoir when the device is *in vivo.* The reservoir caps for each of the reservoirs are designed to open at specified times, thereby delivering the reservoir content sequentially at pre-specified times. These reservoir caps may be independently disintegrated or permeabilized or groups of the reservoir caps can be actuated simultaneously. This reservoir opening may be passively controlled through the disintegration of the reservoir cap. In a passive control system for example, the timing can be controlled by selecting the reservoir cap dimension, composition, and structure. Simultaneous actuation of the reservoir contents can be obtained by covering multiple reservoirs with reservoir caps of identical dimension, composition, and structure that will release the contents of different reservoirs at the same time.

The compositions of the reservoir caps may be selected from materials that will disintegrate in response to an environment existing *in vivo* in the patient or in response to a component contained in the reservoir. Examples of environmental conditions include, but are not limited to temperature, water, an electrolyte, and enzymes. Alternatively, the material of the reservoir cap may also be selected so that it will disintegrate when exposed to a form of energy that is applied to the patient from an external source or implanted internal source, such as acoustic (audible or ultrasonic) energy, magnetic energy, electromagnetic radiation (e.g., UV, visible, IR light, RF energy, or X-ray).

As used herein, the term "disintegrate" refers to degrading, dissolving, rupturing, fracturing or some other form of mechanical failure, as well as fracture and/or loss of structural integrity of the reservoir cap due to a chemical reaction or phase change (e.g., melting or transitioning from a solid to a gel), unless a specific one of these mechanisms is indicated. Hydrolytic decomposition is a preferred form of disintegration.

In preferred embodiments, the reservoir caps are selected to dissolve or biodegrade *in vivo,* without any intervention by the patient or caregiver. In one particular embodiment, the reservoir caps are formed of a biocompatible polymer, such as a poly(lactic acid), poly(glycolic acid), or poly(lactic-co-glycolic acid)s, as well as degradable poly(anhydride-*co*-imides), of a composition and thickness designed to disintegrate by hydrolysis in a prescribed timeframe, releasing the contents of the reservoir.

As used herein, the term "permeabilize" is used broadly to include without limitation some form of physical change that does not alter the chemical composition or dry mass of the membrane but changes the capability of the membrane to contain the reservoir contents. In a preferred embodiment of permeabilization, the polymer swells, changing its porosity without decomposition so that the reservoir contents are in contact with fluid external to the device and are releasable through the resulting open pore structure.

In a preferred embodiment, a discrete reservoir cap completely covers one of the reservoir's openings. In another embodiment, a discrete reservoir cap covers two or more, but less than all, of the reservoir's openings.

Representative examples of reservoir cap materials include polymeric materials and various types of semi-permeable membranes, and non-polymeric materials. In a preferred embodiment, the reservoir caps are non-porous and are formed of a bioerodible or biodegradable material, known in the art, such as a synthetic polymer, e.g., a polyester (such as PLGA), a poly(anhydride), or a polycaprolactone. The reservoir cap may be a multilayer structure. For example an inner layer may be porous or otherwise control diffusion once the outer, non-permeable layer has disintegrated. The reservoir caps of a single device may be made of different materials, may have different thicknesses, may have different degrees of cross-linking, or a combination thereof, for the purpose of opening different reservoirs at different times relative to one another.

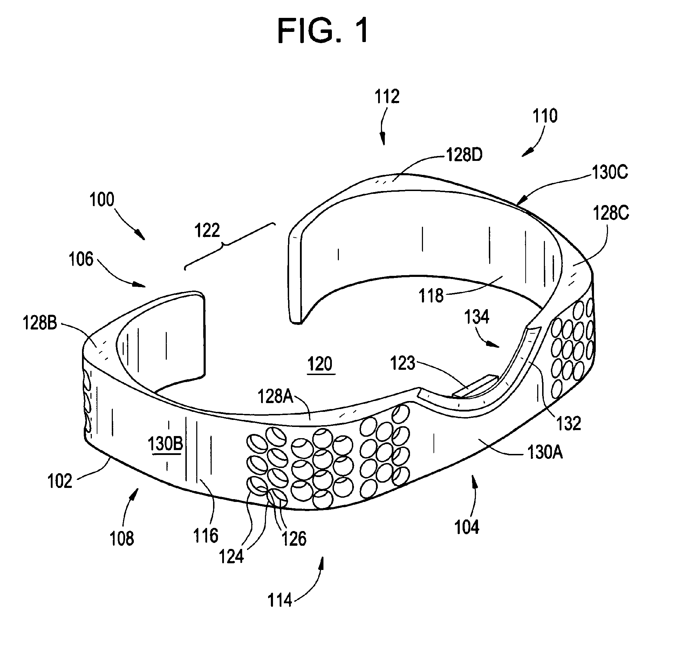

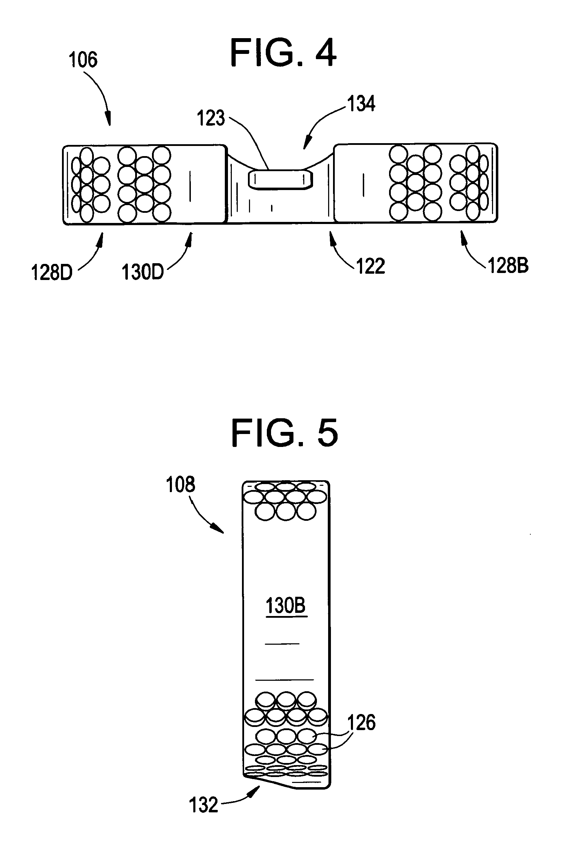

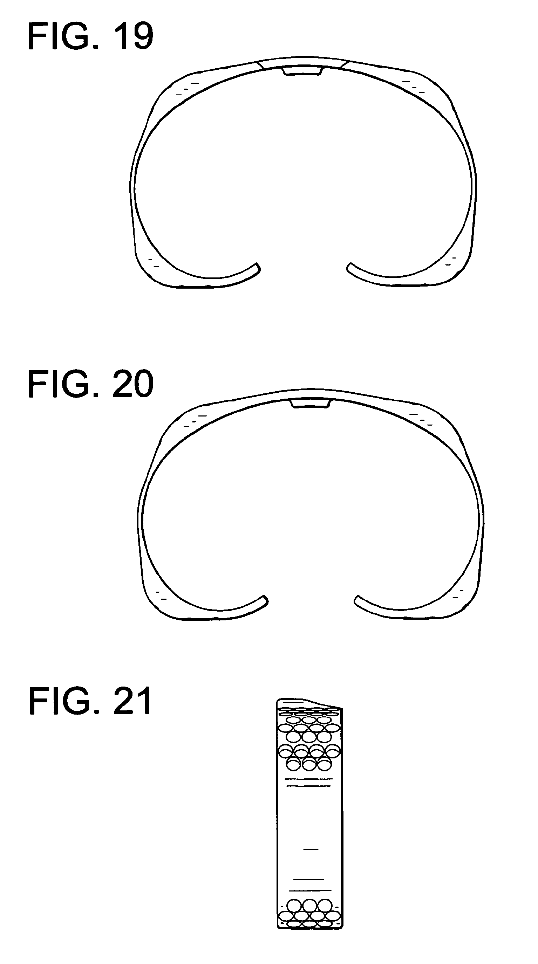

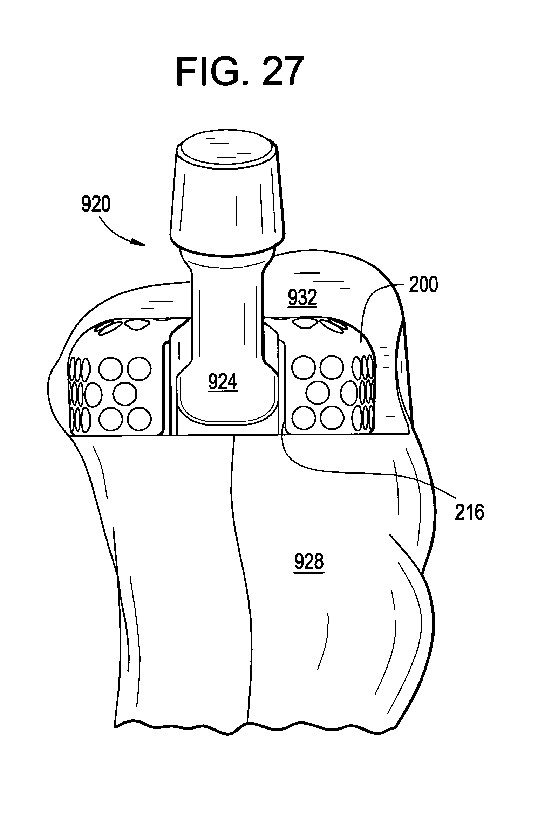

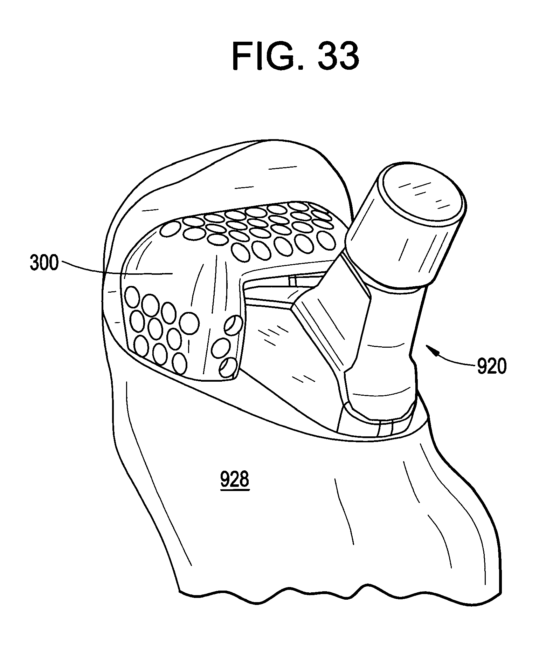

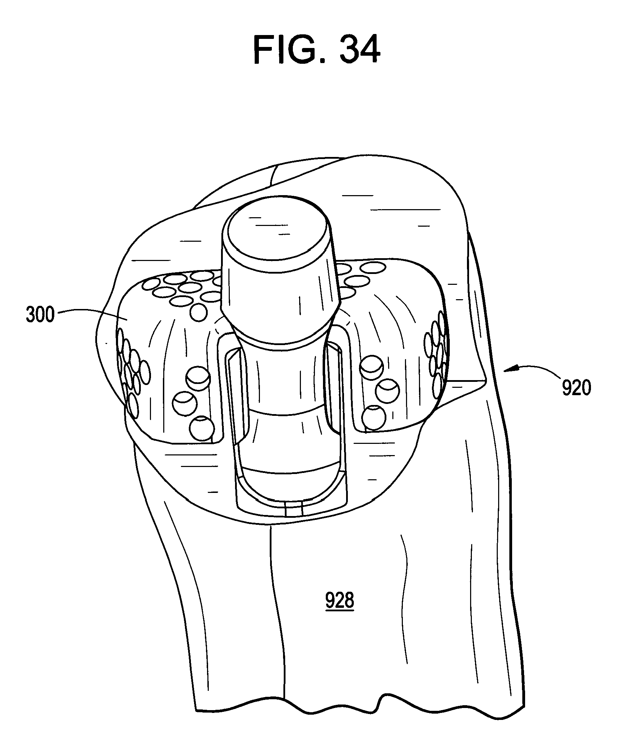

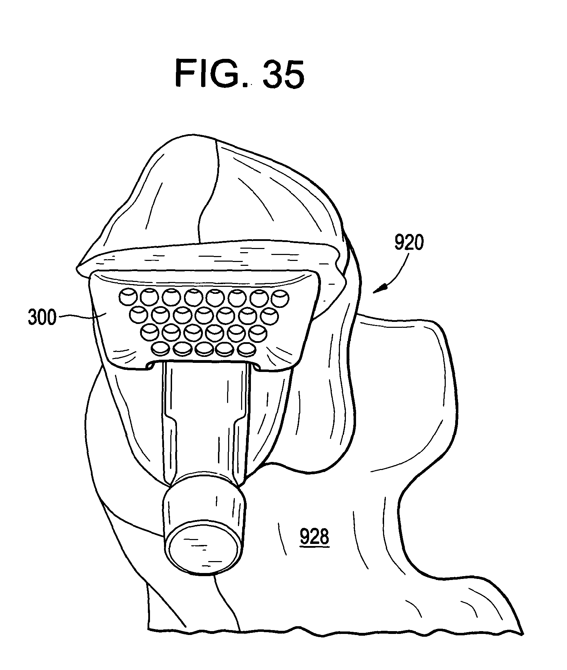

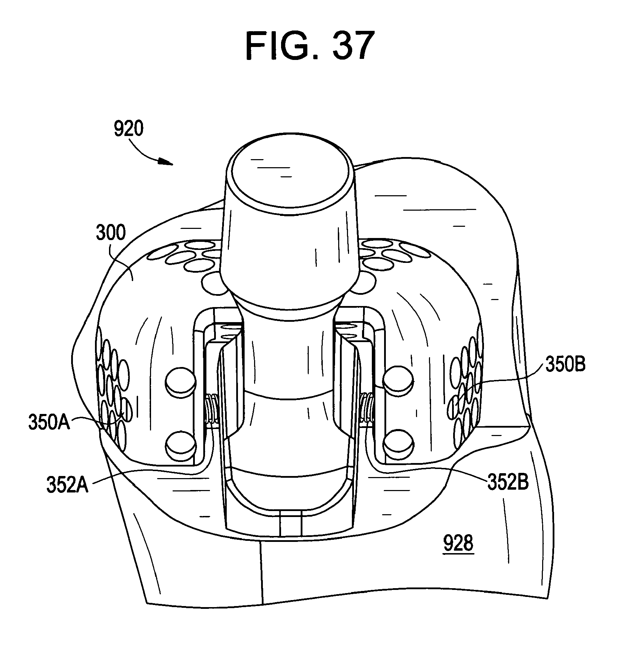

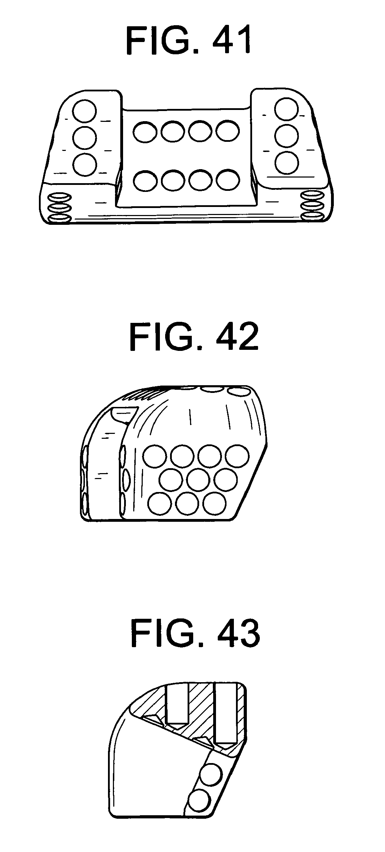

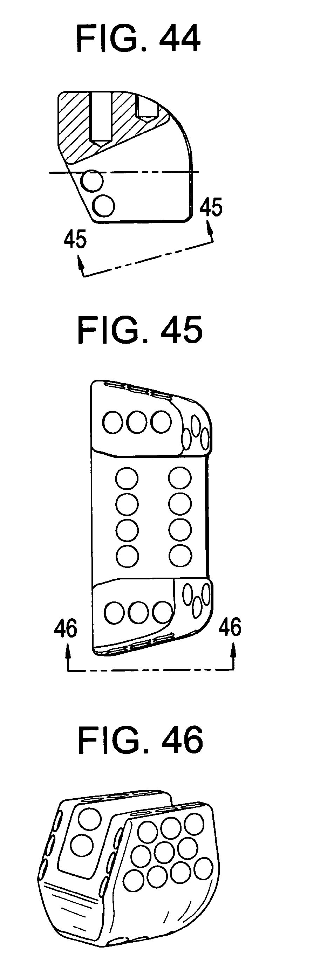

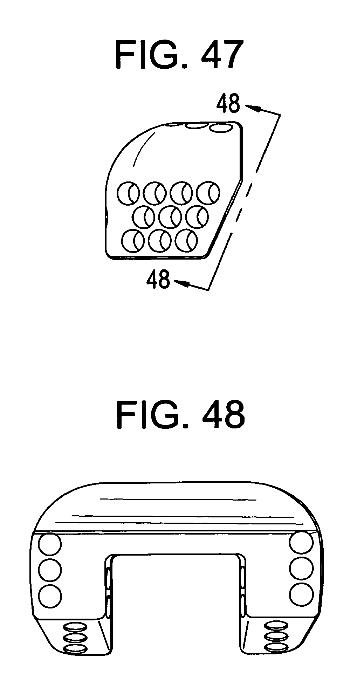

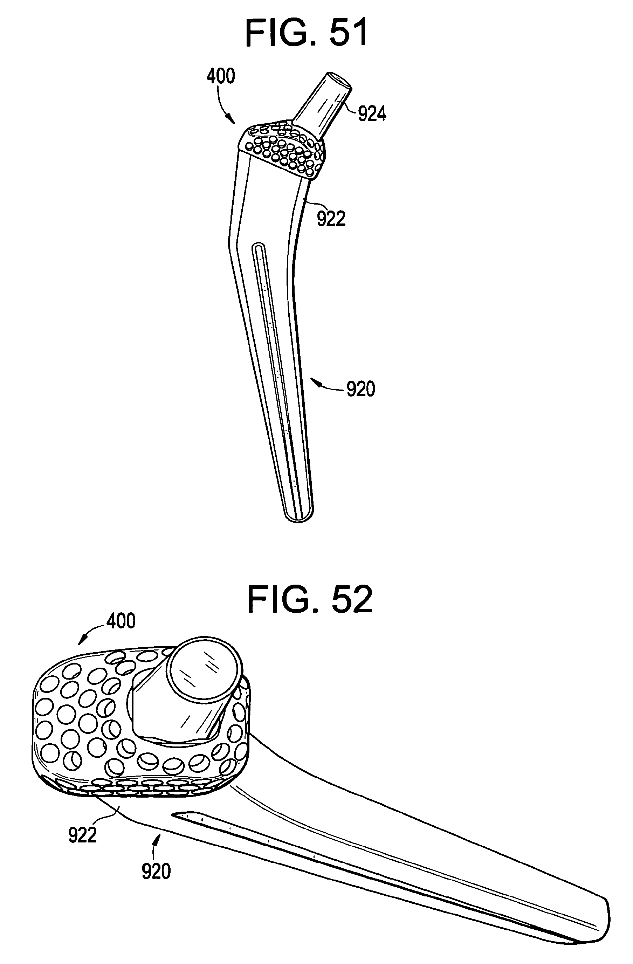

Illustrative, non-limiting examples of the drug delivery devices are described with reference to **FIGS. 1-58****.** In these figures, the drug formulation is not shown in the reservoirs in order to more clearly illustrate the device body and reservoirs.

### Device for Knee Prosthetic

**FIGS. 1-21** show one embodiment of a drug delivery device **100** adapted for use with a standard knee replacement prosthetic device. The device **100** is designed to be affixed to portion of the knee prosthesis and/or an adjacent surgically prepared bone section, such that the adjacent ligaments and tendons are accommodated. For example, the knee prosthesis may be implanted on a surgically prepared area between the tibia and the femur, and the drug delivery device may be implanted on a tibial portion of the knee prosthesis. Once implanted, the device **100** permits local drug delivery to the implantation site of the knee prosthesis. For example, the drug delivery device **100** may release an antibiotic over the course of several weeks or months to reduce the likelihood of infection associated with the knee prosthesis implantation. The device **100** may be secured with reference to the knee prosthesis to prevent relative movement between the device **100** and the knee prosthesis.

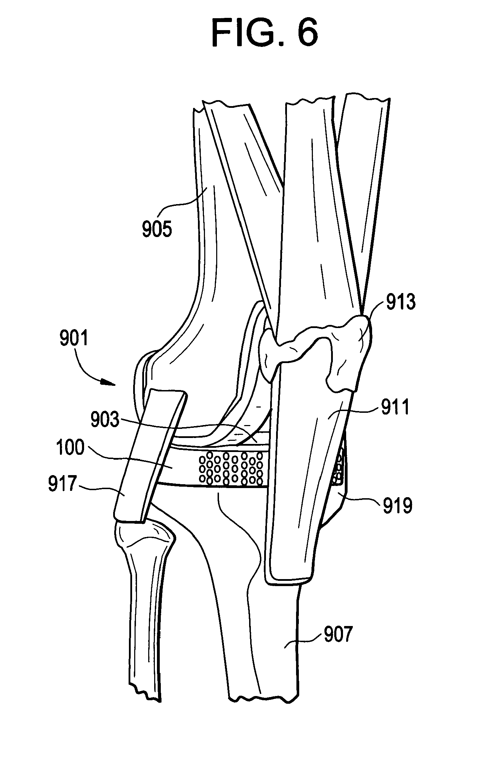

As shown in **FIGS. 1-5****,** which are perspective, top, front, back, and left side views of the device **100**, respectively, the device includes a generally C-shaped body **102** having a front portion **104,** a back portion **106**, a left portion **108,** a right portion **110**, a top side **112**, and a bottom side **114**. The directional designations correspond to the orientation of the device once implanted about the knee prosthesis. As shown in **FIGS. 6-9****,** the device **100** is adapted to be implanted about the tibial portion **903** of the knee prosthesis 901, with the top side **112** of the device **100** positioned adjacent to the femur 905, the bottom side **114** positioned adjacent to the tibia **907,** the front portion **104** positioned about the articular surface **909** adjacent to the patellar tendon **911** and the patella **913**, the back portion **106** positioned adjacent to the posterior cruciate ligament (PCL) **915** , the left portion **108** positioned adjacent to the lateral collateral ligament (LCL) **917,** and the right portion **110** positioned adjacent to the medial collateral ligament (MCL) **919.**

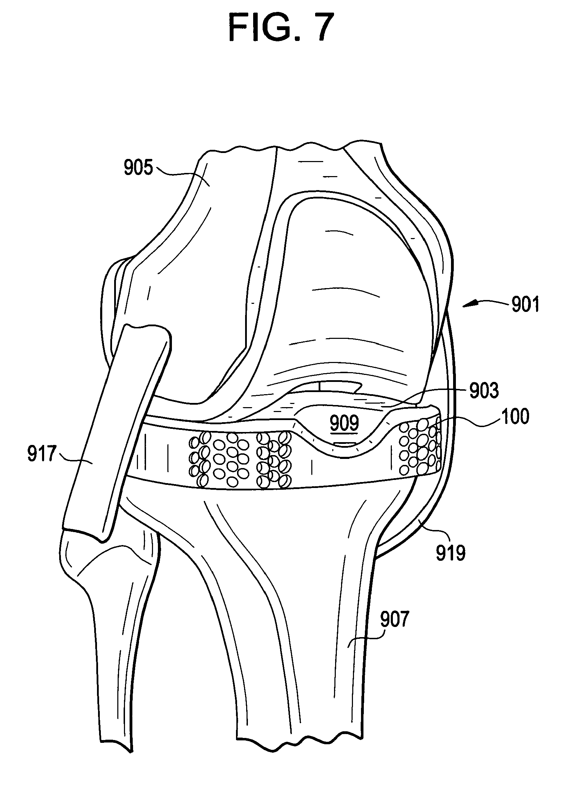

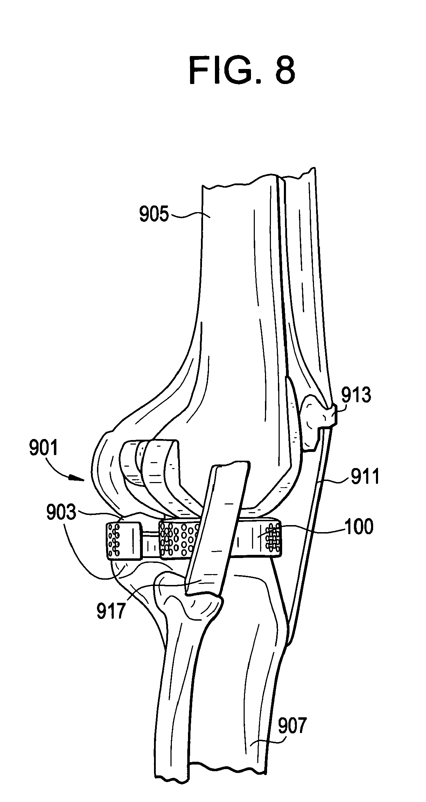

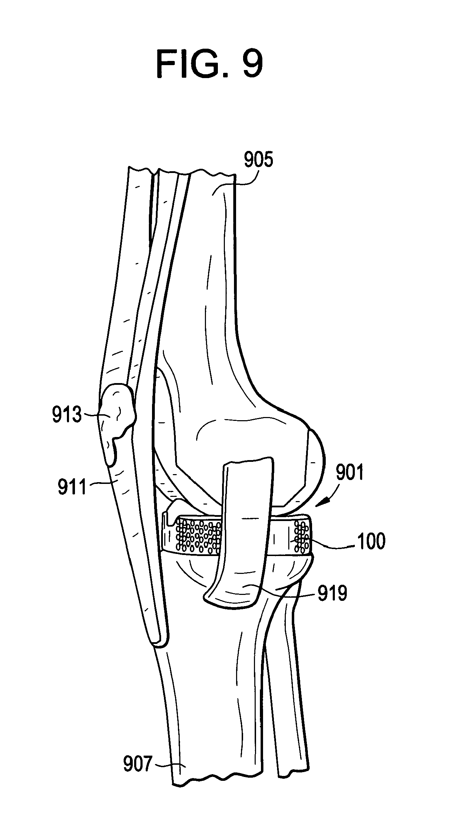

More specifically, **FIG. 6** is a front perspective view of the implanted device **100**, illustrating the front portion **104** of the device **100** positioned behind the patellar tendon 911 and the left portion **108** of the device **100** positioned behind the LCL **917.** **FIG. 7** is a front perspective view of the implanted device **100,** with the patella and the patellar tendon removed, illustrating the front portion **104** of the device **100** positioned adjacent to the articular surface **909**. **FIG. 8** is a left rear perspective view of the implanted device **100**, illustrating the left portion **108** of the device **100** positioned behind the LCL **917.** Although the PCL is not shown, when the device **100** is so implanted the back portion **106** of the device **100** may be out of contact with the PCL. **FIG. 9** is a right side view of the implanted device **100** illustrating the right portion **110** of the device **100** positioned beneath the MCL **919.**

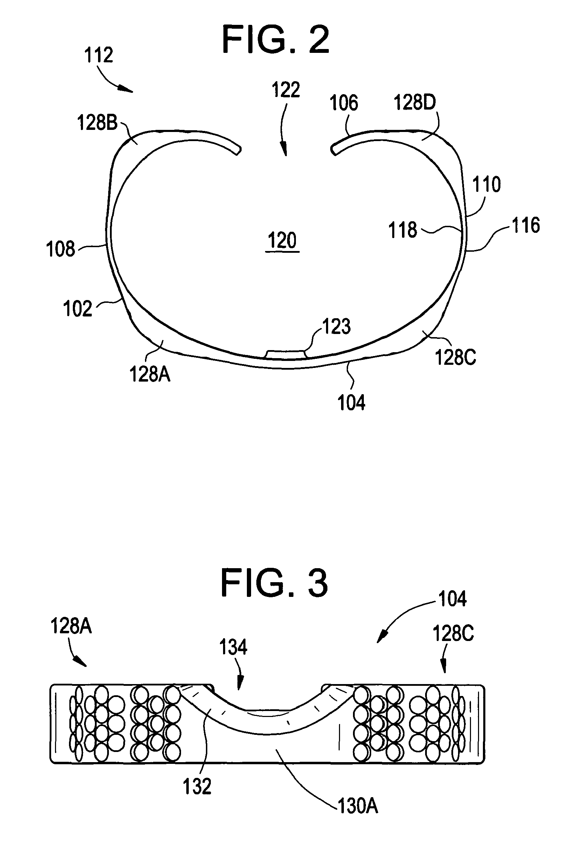

With reference back to **FIGS. 1** and **2****,** The body **102** includes an outer surface **116** and an inner surface **118**. The inner surface **118** defines an aperture **120** that is sized and shaped for receiving the tibial portion **903** of the knee prosthesis **901**. The aperture **120** extends through the body **102** along a length of the inner surface **118** to permit accommodating the tibial portion **903** of the knee prosthesis **901** within the aperture **120.** The body **102** surrounds and substantially encloses the aperture **120** except at a back gap area **122** positioned on the back portion **106,** which permits associating the device **100** with the tibial portion **903.** More specifically, the body **102** may be adapted to slide over the tibial tray and the articular surface **909.**

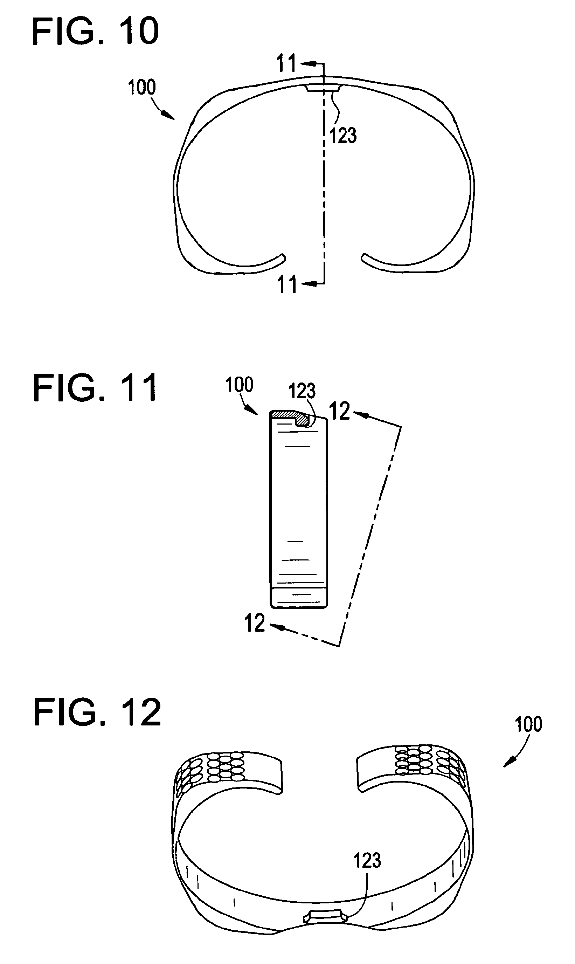

The inner surface **118** may include a locking pin **123** adapted to lock the body **102** about the tibial portion **903.** The locking pin **123** may be sized and shaped to mate with a groove in the tibial portion **903**. For example, the locking pin **123** may be positioned on the inner surface **118** of the front portion **104** of the device **100,** in which case the groove (not shown) may be formed on the articular surface **909** of the knee prosthesis **901**. An embodiment of the locking pin **123** can be seen in **FIGS. 10-12****,** where **FIG. 11** is a cross-sectional view of the device **100** taken along line C-C of **FIG. 10,** and **FIG. 12** is a rear perspective view of the device **100** taken along line D-D in **FIG. 10**. Additionally, the body **102** also may be epoxied to the articular surface in addition or as an alternative to the locking pin **123,** and/or the body **102** may be secured to the knee prosthesis using any of the fixation means described above.

The outer surface **116** has a number of openings **124** formed through it. The openings **124** may be in communication with a number of discrete reservoirs **126** formed in the device body **102**. The reservoirs **126** may store one or more drug formulations that can be released through the openings **124** into the implantation site. In one embodiment, each reservoir **126** may be associated with one opening **124** in the outer surface **116.** The orientation of the openings **124** along the outer surface **116** may permit releasing the drug formulation in radially outward directions. The reservoirs **126** are also referred to herein as "wells" or "pockets".

In embodiments, the reservoirs **126** may be located in groupings or arrays. In some embodiments, the reservoirs **126** may be located in lobe regions **128** positioned about the device body **102**. For example, in the illustrated embodiment, the reservoirs **126** are located within four lobe regions **128**, including a front left lobe region 1**28A,** a rear left lobe region **128B,** a front right lobe region **128C**, and a rear right lobe region **128D.** The front left lobe region 128A is positioned at the intersection of the front portion **104** and the left portion **108** of the device body **102**. The rear left lobe region **128B** is positioned at the intersection of the left portion **108** and the back portion **106** of the device body **102**. The front right lobe region **128C** is positioned at the intersection of the front portion **104** and the right portion **110** of the device body **102**. The rear right lobe region **128D** is positioned at the intersection of the right portion **110** and the back portion 106 of the device body **102.**

In each of these lobe regions **128**, the body **102** may have a number of reservoirs **126**, and the outer surface **116** of the body **102** may have a number of openings **124** that permit releasing the drug formulation from the corresponding reservoirs **126** into the implantation site. The size, number, and position of reservoirs **126** may be selected to permit storing and releasing a predefined amount of the drug formulation from a given lobe region **128** or from the body **102** has a whole. The size, number, and position of reservoirs **126** may also be selected based on the dimensions of the lobe region **128**. More specifically, about the lobe region **128** the body **102** may have a thickness in the radial direction that is selected to accommodate the reservoirs **126** within an interior of the body 102. (The thickness also may be selected based on the available space about the knee prosthesis, although a corresponding region about the human knee is generally sufficiently sized to not present a design limitation.)

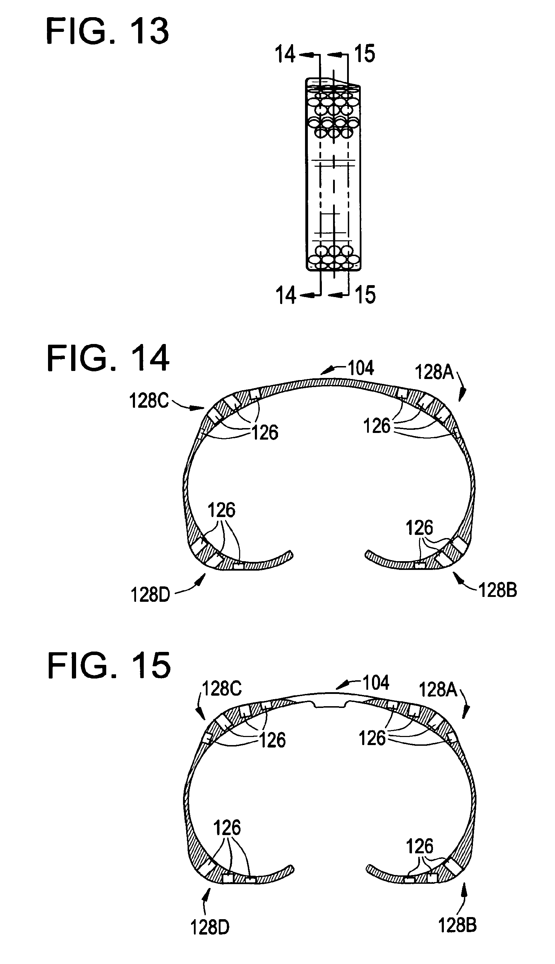

For example, **FIG. 14** is a cross-sectional view of the device **100**, taken along the line A-A in **FIG. 13,** and **FIG. 15** is a cross-sectional view of the device **100,** taken along the line **B-B** in **FIG. 13**. As shown, each lobe region **128** may have a thickness that varies or tapers about the periphery of the body **102** from a relatively wider central point to relatively narrower outer points. Due to the difference in thickness across the lobe region **128,** the interior of the device body **102** may have a varying amount of space for accommodating the reservoirs **126**, and therefore the reservoirs **126** may have varying cross-sectional shapes and sizes, with reservoirs **126** that are centrally positioned being relatively larger than reservoirs **126** positioned about outer edges of the lobe region **128.** For example, the device **100** may include reservoirs **126** sized to store a total volume of about 2 mL of the drug formulation, and the openings **124** have diameters of about 3 mm for releasing the drug formulation from the reservoirs **126**. In other embodiments, the lobe regions **128** may be coated in a drug-eluting coating, in addition or as an alternative to the reservoirs **126**.

In one embodiment, the body **102** may also include a number of low influence regions **130.** The low influence regions **130** may be configured to minimize or avoid irritating a tendon or a ligament. For example, the outer surface **116** of the body **102** may be relatively smooth in the low influence regions **130**. The thickness of the body **102** in the radial direction may be relatively narrow in the low influence regions **130**. The body **102** may also be substantially free from reservoirs **126** in the low influence regions **130,** and the outer surface **116** may not include openings **124.**

The low influence regions **130** may be positioned about the body **102** spaced apart from the lobe regions **128.** Specifically, the low influence regions **130** may be positioned such that when the device **100** is implanted about the knee prosthesis, the low influence regions **130** become positioned adjacent to the tendons and ligaments and the lobe regions **128** become positioned spaced apart from the tendons or ligaments, so that irritation of the tendons and ligaments is reduced or eliminated.

In the illustrated embodiment, the low influence regions **130** include a patellar tendon low influence region **130A**, a LCL low influence region 130B, a MCL low influence region **130C,** and a PCL low influence region **130D**. The patellar tendon low influence region **130A** is positioned on the front portion **104** of the body **102** between the front left lobe region **128A** and the front right lobe region **128C**. The LCL low influence region **130B** is positioned on the left portion **108** of the device body **102** between the front left lobe region **128A** and the rear left lobe region **128B.** The MCL low influence region **130C** is positioned on a right portion **110** of the body **102** between the front right lobe region **128C** and the rear right lobe region **128D.** The PCL low influence region **130D** is positioned on the back portion **106** of the body **102** between the rear left lobe region **128B** and the rear right lobe region **128D.** In each of these low influence regions **130**, the body **102** is relatively narrower in the radial direction, the outer surface **116** is relatively smooth, and the body **102** is substantially free from reservoirs **126**. Additionally, the patellar tendon low influence region **130A** may include a tapered area **132** over which the thickness of the body **102** further reduces to become a front cut-away area **134.** The tapered area **132** and front cut-away area **134** may further accommodate the patellar tendon **911** and the patella **913.** Similarly, the back gap area **122** further provides a relief area for accommodating the PCL. When the device **100** is implanted, the patellar tendon low influence region **130A** becomes positioned adjacent to the patellar tendon **911**, the LCL low influence region **130B** becomes positioned adjacent to the LCL **917**, the MCL low influence region **130C** becomes positioned adjacent to the MCL **919,** and the PCL low influence region **130D** becomes positioned adjacent to the PCL **915.**

In alternative embodiments, the low influence regions **130** may include an array of sufficiently small reservoirs and openings, such as micro-reservoirs and associated micro-openings. The micro-reservoirs may contain one or more drug formulations or other substances, which may be released through the micro-openings without substantially irritating the adjacent tendons or soft tissue.

The body **102** may be of a unitary construction. The body **102** may formed of a biocompatible material, such as a metal, a polymer, a ceramic, or a combination thereof as described herein. The biocompatible material may or may not be biodegradable or bioerodible. For example, the body **102** may be formed from a biodegradable or bioerodible polymer.

In one embodiment, the body **102** may be formed of, for example, a biocompatible metal, a non-biodegradable polymer of sufficiently rigidity, a non-biodegradable ceramic, a degradable polymer, or a combination thereof. In some embodiments, the body **102** may be formed from a core covered in a drug-eluting coating. The coating may be a blend of a polymer and a drug, such that the drug is released from the coating *in vivo.* In embodiments in which the body **102** has a core, the coating may cover the lobe regions **128** and the reservoirs **126** may be omitted. Alternatively, the body **102** may have the reservoirs **126** and the drug-eluting coating.

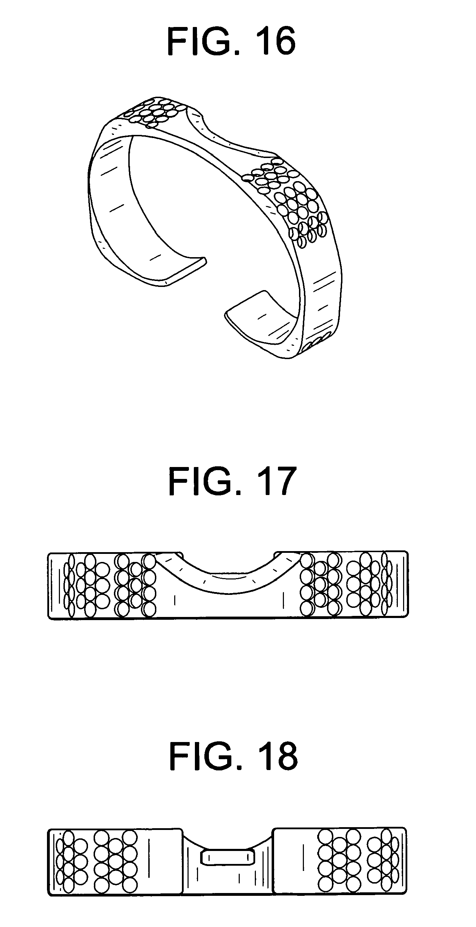

The body **102** may also have a variety of different sizes to accommodate conventional knee prosthesis of varying sizes. For example, **FIGS. 16-21** are front, perspective, rear, top, left side and bottom views, respectively, of an embodiment of the drug delivery device shown in **FIG. 1**, illustrating example dimensions of the drug delivery device. To accommodate knee prosthesis of other sizes, the body **102** may be made relatively smaller or larger, such as by varying one or more dimensions of the device **100.** It should be noted that devices **100** of different sizes may have different locations, sizes, and numbers of reservoirs to accommodate the desired volume of drug formulation while maintaining the mechanical integrity of the device.

The device **100** may be incorporated into a knee replacement procedure, such as a total knee replacement arthroplasty (TKA) procedure, with relative ease. The device 100 may permit controlled release of any number of drug formulations, including antibiotics, into the implantation site. Further, the device may not impinge against the knee prosthesis or adjacent anatomy upon movement.

### Device for Hip Prosthetic

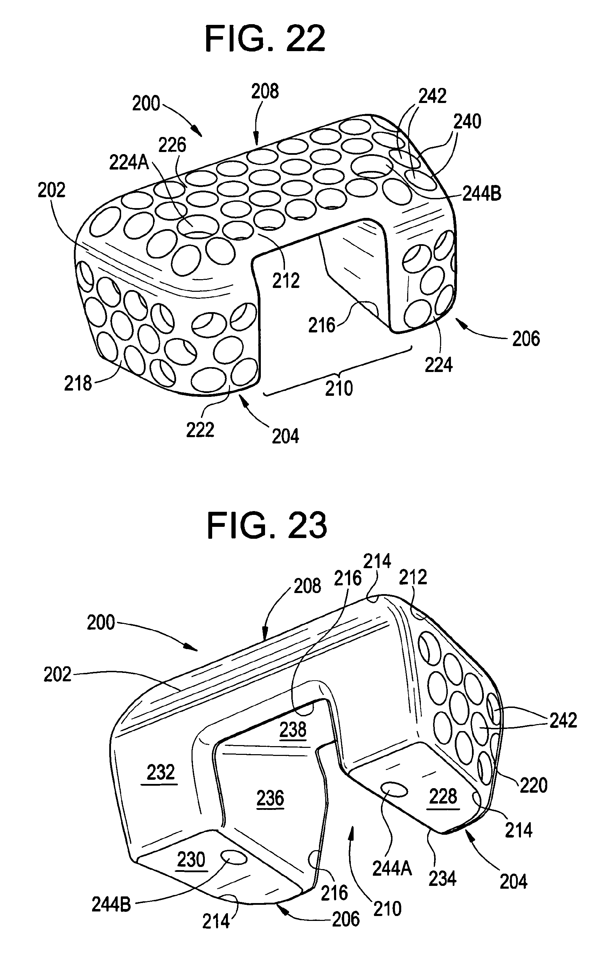

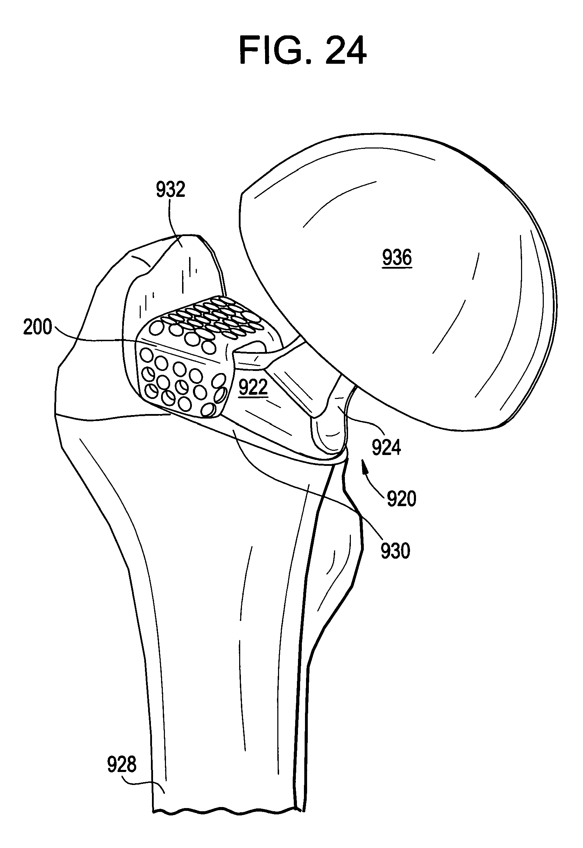

**FIGS. 22-30** show embodiments of a drug delivery device **200** for use with, for example, a standard hip replacement prosthesis. In embodiments, the drug delivery device **200** is designed to be positioned adjacent to a hip stem of the hip prosthesis. More specifically, the drug delivery device **200** may be implanted between a surgically prepared portion of the femur and the hip stem of the implanted hip prosthesis. Once implanted, the drug delivery device **200** permits local drug delivery to the implantation site of the hip prosthesis. For example, the drug delivery device **200** may release an antibiotic over the course of several weeks or months, to reduce the likelihood of infection associated with the implantation of the hip prosthesis. The device **200** may be secured with reference to the hip prosthesis to prevent relative movement between the device **100** and the hip prosthesis. The drug delivery device **200** may also be suited for use with other prosthetic implants or parts of the anatomy in general. As shown in **FIGS. 22-23**, which are upper and lower perspective views of the drug delivery device **200,** the device **200** includes a generally bridge shaped or arcuate body **202.** More specifically, the body **202** includes a front pillar **204,** a rear pillar **206,** and an intermediate cross piece **208** extending between the front pillar **204** and the rear pillar **206** on an upper portion of the device **200**. Below the intermediate cross piece **208**, an aperture or slot **210** is defined on a lower portion of the device **200.** Thus, the device **200** is shaped to be implanted about a hip prosthesis **920,** as shown in **FIGS. 24-30****.** As shown, the hip prosthesis **920** generally includes a hip stem **922**, a neck **924,** and a head or ball **926.** The head **926** may be associated with an acetabular cup **936,** which is designed to be positioned in a socket of the pelvis. The hip stem **922** may be an unmodified, tapered hip stem. The hip prosthesis **920** is normally implanted on a section of the femur **928** that has been surgically prepared, such as by cutting the femur **928** to remove at least a portion of the femoral head, thereby forming a lower cut surface **930** and a lateral cut surface **932.** Typically, the cut surfaces **930, 932** are relatively planar and form an included angle of greater than 90°, such as an angle of about 146°, although other angles are possible. The hip prosthesis **920** is then implanted in the femur **928** with the hip stem **922** extending through the lower cut surface **930,** the neck **924** protruding from the hip stem **922** toward the pelvis, and the head **926** positioned on an end of the neck **924** for association with the acetabular cup 936 and the pelvis.

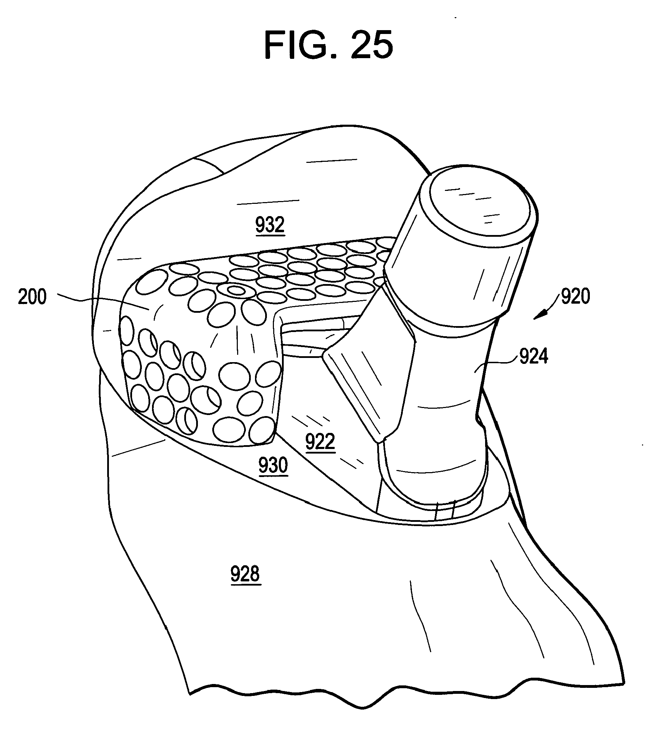

After the hip prosthesis **920** is so implanted, the drug delivery device **200** may be implanted about the hip prosthesis **920.** FIG. 24 is a perspective view of the drug delivery device **200** positioned about a portion of an implanted hip prosthesis **920,** such as on the surgically prepared portion of the femur **928** in a space formerly occupied by the femoral head. The front pillar **204** may be positioned in front of the hip stem **922,** the rear pillar **206** may be positioned behind the hip stem **922,** and the intermediate cross piece **208** may extend above the hip stem **922** adjacent to the neck **924**. The aperture **210** may be accommodate the hip stem **922** as shown in **FIG. 25****,** which is another perspective view of the drug delivery device **200** positioned about a portion of an implanted hip prosthesis **920**, illustrating the hip stem **922** with the head **926** removed. More specifically, the aperture **210** may receive the implant in confronting relation around three sides of the hip stem **922.**

It should be noted that directional designations, such as front, back, left, right, forward, rearward, upper, lower, and lateral, among others, are provided for explanatory purposes. These directions correspond to the orientation of the recited component with reference to a left leg positioned with a left foot on the floor. Of course, the relative orientation of the hip prosthesis **920** and the drug delivery device **200** will vary with movement of the leg. Therefore, these directional designations are not intended to limit the disclosure. Further, the device **200** is illustrated and described with reference to the left leg, although the device **200** may be suited for use with either leg merely by rotating the device 180°. In such cases, the directional designations referenced above are 180° out of phase. The device **200** may also be suited for implantation about other portions of the anatomy or prosthetic implants.

With reference back to **FIGS. 22-23****,** the device body **200** is generally bounded by an outer or exposed surface **212**, inner or occluded surfaces **214**, and a partially occluded surface **216.** The exposed surface **212** is outwardly directed and is adapted to permit releasing a drug formulation into a targeted area, such as the implantation site of a hip prosthesis **920.** The occluded surfaces **214** are inwardly directed and are adapted to be positioned in confronting relation to a surgically prepared bone section, such as one or more of the cut surfaces **930, 932** of the femur **928.** The partially occluded surface **216** is adapted to be positioned about or in close proximity to a portion of the prosthetic implant **920,** such as the hip stem **922** of the hip prosthesis **920.**

The exposed surface **212** is best seen in **FIG. 22****,** and generally includes a front face **218** of the front pillar **204,** a rear face **220** of the rear pillar **206,** right side faces **222, 224** of the front and rear pillars **204, 206,** and an upper face **226** extending along the front pillar **204,** the intermediate cross piece **208,** and the rear pillar **206.** The body **202** is generally curved at intersections of these faces such that the exposed surface **212** is relatively free of sharp edges. The exposed surface **212** is "exposed" in the sense that, once the device **200** is implanted in the body, the exposed surface **212** is generally exposed to the implantation environment, such that the drug formulation released through the exposed surface **212** may have the intended effect at the implantation site.

The occluded surfaces **214** are best seen in **FIG. 25****.** The occluded surfaces **214** generally includes lower faces **228, 230** of the front and rear pillars **204, 206,** and a left side face **232** extending along the front pillar **204,** the intermediate cross piece **208,** and the rear pillar 206. The occluded surfaces **214** are "occluded" in the sense that, once the device **200** is implanted in the body, the occluded surfaces **214** are positioned in confronting relation to the surgically prepared bone sections, such as the lower and lateral cut surfaces **930, 932** of the femur **928.**

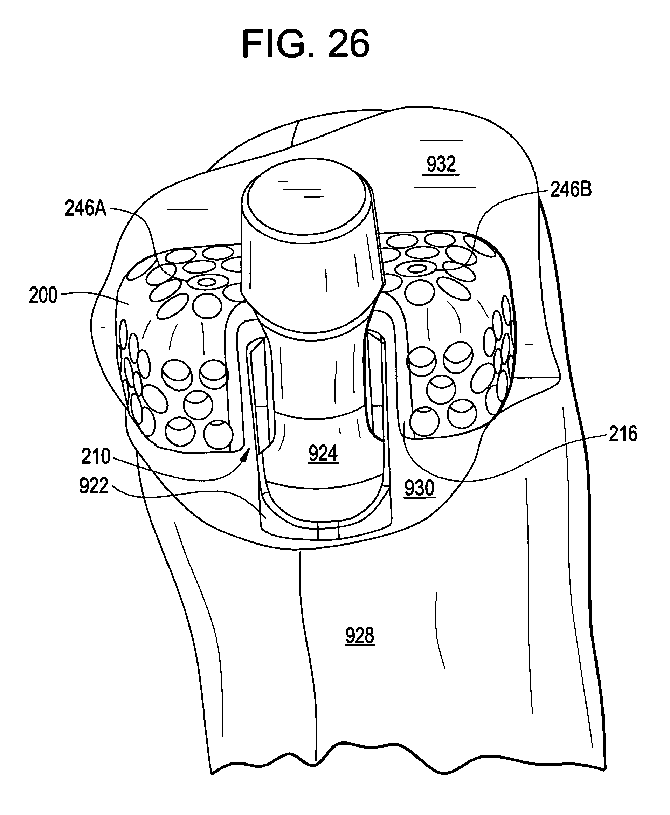

The partially occluded surface **216** includes a rear face **234** of the front pillar **204,** a front face **236** of the rear pillar **206,** and a lower face **238** of the intermediate cross piece **208.** The partially occluded surface **216** is "partially occluded" in the sense that, once the device **200** is implanted in the body, the partially occluded surface **216** is adjacent to and in close proximity to a portion of the prosthetic implant **920,** such as the hip stem **922,** but is at least slightly spaced apart from contact with the portion of the prosthetic implant **920**. Such positioning of the partially occluded surface **216** is best seen in **FIGS. 26-27****,** which are side views of the device **200** implanted adjacent to the hip stem **922** with the head **926** removed for illustrative purposes.

The device **200** may be adapted to release a drug formulation into the implantation site once implanted, such as through the exposed surface **212**. With reference back to **FIG. 22****,** the exposed surface **212** may have a number of openings **240** formed through it. The openings **240** may be in communication with a number of discrete reservoirs **242** formed in the device body **202.** The reservoirs **242** may store one or more drug formulations that can be released through the openings **240** into the implantation site. The drug formulation is not shown for clarity. In one embodiment, each reservoir **242** may be associated with one opening **240** in the exposed surface **212.** The orientation of the openings **240** along the exposed surface **212** may permit releasing the drug formulation in radially outward directions. Thus, when the device **200** is implanted, the drug formulation may be released from the reservoirs **242** through the exposed surface **212** via the openings **240 a**nd into tissues and fluids at the implantation site.

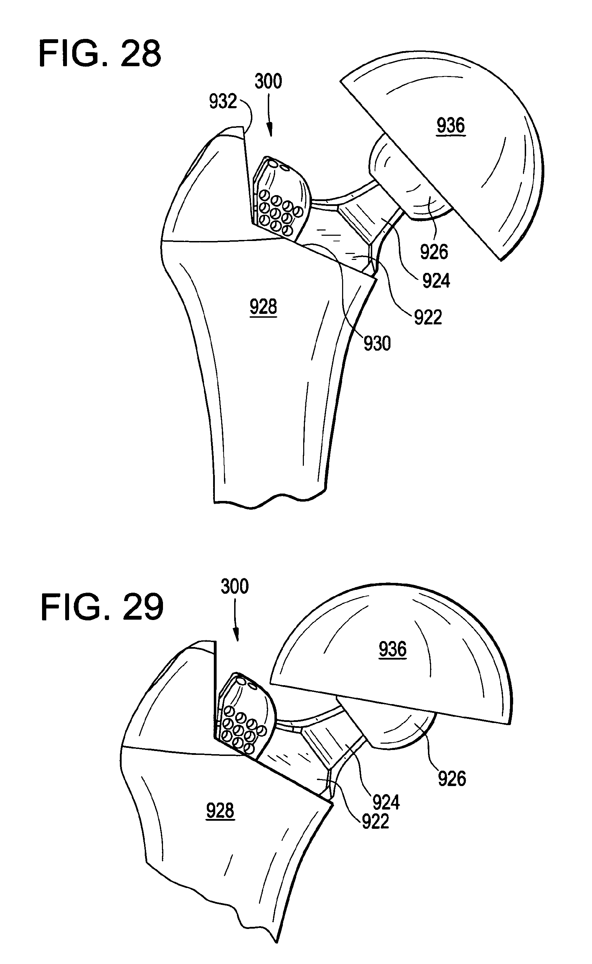

The device **200** may be sized for use with commercially available hip prostheses of various sizes. More specifically, the pillars **204, 206** may be sized such that once the device **200** is implanted, the upper face **226** is even with or lower than the remaining portion of the femur **928.** Such sizing reduces the likelihood of the device **200** interfering with remaining portions of the anatomy, such as adjacent muscles, tendons, and ligaments. The aperture **210** may also be sized to accommodate hip stems **922** of various dimensions and configurations, with the partially occluded surface **916** being spaced apart from the hip stem **922** to prevent mechanical interaction between the device **200** and the hip stem **922**. The upper face **226** of the body **200** may slope slightly away from the left side face **232,** so that the acetabular cup **936** may not impinge against the drug delivery device 200 as the leg is rotated. For example, the average abduction angle permitted by an implanted hip prosthesis **920** is about 25 degrees, while the device **200** may permit rotation of as much as 45 degrees without impingement occurring. Such a configuration is shown in **FIGS. 28-29****,** which are front views of another embodiment of a drug delivery device **300** (described in further detail below) illustrating the device **300** implanted adjacent to a portion of an implanted hip prosthesis **920,** with the acetabular cup **936** in a non-rotated position and rotated position, respectively, the acetabular cup **936** not impinging against the drug delivery device **300** when in the rotated position despite a large degree of rotation about the head **926.**

Because the cut surfaces **930, 932** are generally cut in a roughly planar configuration during the surgical preparation, the occluded surfaces **214,** including the lower faces **228, 230** of the pillars **204, 206** and the left side surface **232,** may be generally planar. Each lower face **228, 230** may form an angle with the left side face **232**. The angle may substantially correspond to the included angle between the lower cut surface **930** and the lateral cut surface **932,** so that the occluded surfaces can be placed in confronting relation to the surgically prepared bone section. For example, the angle may be about greater than 90 degrees, such as at about 146 degrees in some embodiments, although other configurations are possible.

The size and shape of the device **200** may permit housing the reservoirs **242**, and therefore the drug formulation, within the body **202.** The reservoirs **242** may have varying cross-sectional shapes and sizes depending on the position on the body **202,** as varying amount of space may be available for accommodating the reservoirs **242** within the body **202**. For example, each reservoir **242** may have a diameter of about 3 mm and a depth varying from about 1.35 mm to about 9 mm, depending on the location on the body **202.** In total, the reservoirs **242** may be sized to store a total volume of about 2 mL of the drug formulation, which may be an antibiotic. The reservoirs **242** may be in communication with the openings **240** located about the exposed surface **212,** such as on the front face **218** of the front pillar **204**, and the rear face **220** of the rear pillar **206**, the right side faces **222,** 224 of the pillars, and the upper face **226**. In the embodiment shown, reservoirs **242** are not provided on the occluded surfaces **214** or the partially occluded surface **216.** For example, the occluded surfaces **214** may be relatively free from reservoirs **242** to avoid releasing drug formulation onto adjacent portions of the of the femur **928,** which may be irritated. The partially occluded surface **216** may also be relatively free from reservoirs **242,** so that the drug formulation is generally released into the implantation site instead of onto the hip stem **922.** However, the occluded and/or partially occluded surfaces **214, 216** may have reservoirs, such as microreservoirs, in other embodiments.

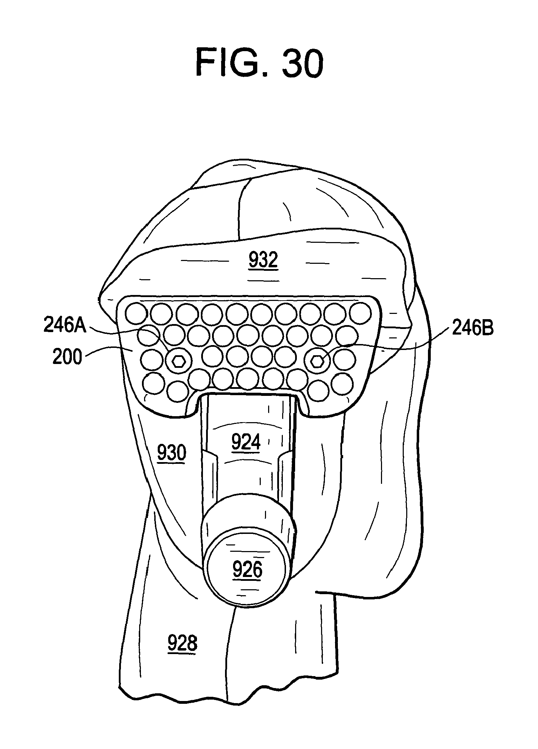

The device **200** may be secured to the surgically prepared bone section and/or the implanted prosthesis in a variety of manners. In the embodiment illustrated in **FIGS. 22-30**, for example, the device **200** is adapted to be attached to the surgically prepared portion of the femur with a number of screws. The body **202** may include a number of screw holes **244** that extend substantially vertically through the device body **202,** and a corresponding number of screws **246,** such as conventional bone screws, may be positioned in the screw holes **244**. As shown in **FIGS. 22-23****,** for example, a front screw hole **244A** may extend through the front pillar **204** from the upper face **226** through the lower face **228**, and a rear screw hole **244B** may extend through the rear pillar **206** from the upper face **226** through the lower face **230.** As shown in **FIGS. 26** and **30****,** a front screw **246A** may be positioned in the front screw hole **244A** to secure the front pillar **204** to the lower cut surface **930** and a rear screw **246B** may be positioned in the rear screw hole **244B** to secure the rear pillar **206** against the lower cut surface **930**. However, in other embodiments, any number or positioning of screw holes **244** and corresponding screws **246** may be used.

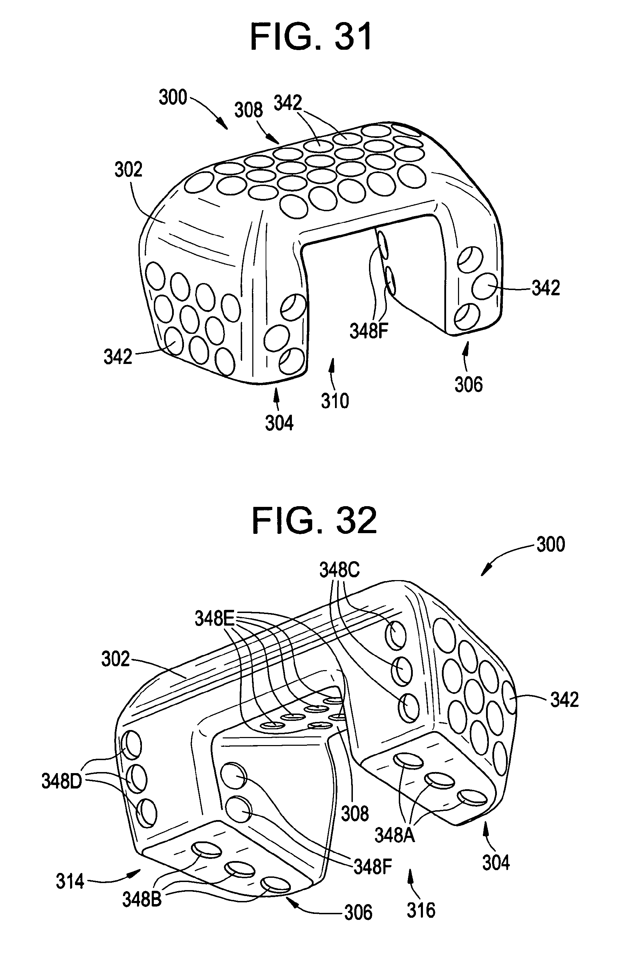

Further, the device may also be secured in other manners. For example, **FIGS. 31-38** illustrate another embodiment of a drug delivery device **300**. Although the device **300** is substantially similar to the device **200,** the device **300** is adapted to be secured with bone cement. More specifically, the body **302** may include a number of cement holes **348** that extend into the device body **302,** and a bone cement, such as conventional bone cement, may be positioned in the cement holes **348**. The cement holes **348** may be formed through the pillars **304, 306** and the intermediate cross piece **308**. The cement holes **348** may be exposed on the occluded surfaces **314 f**or securing the device **300** to the cut surfaces **930, 932.** The cement holes **348** may also be exposed on the partially occluded surface **316** for securing the device **300** to a portion of the implant, such as the hip stem **922** of the hip prosthesis **920**. As shown in **FIGS. 31-32**, for example, a set of front vertical cement holes **348A** may extend into the front pillar 304 from the lower face, and a set of rear vertical cement holes **348B** may extend into the rear pillar **306** from the lower face. Similarly, a set front and rear lateral cement holes **348C, 348D** may extend into the front and rear pillars **304, 306**, respectively, from the corresponding the left side face. A set of intermediate vertical cement holes **348E** may extend into the intermediate cross piece **308** from the lower face, and a set of intermediate lateral cement holes **348F** may extend through the front and rear pillars **304, 306** from the inner faces. Bone cement may be positioned in the front and rear vertical cement holes **348A, 348B** to secure the front and rear pillars **304, 306** to the lower cut surface **930**, in the front and rear lateral cement holes **348C, 348D** to secure the front and rear pillars **304, 306** to the lateral cut surface **932,** in the intermediate vertical cement holes **348E** to secure the intermediate cross piece **308** to the hip stem **922,** and in the intermediate lateral cement holes **348F** to secure the front and rear pillars **304, 306** to the hip stem **922**. However, any number or positioning of cement holes **348** may be used in other embodiments. Although the drug delivery device **300** is generally similar to the drug delivery device **200,** the size and shape of the device **300** may vary slightly to accommodate the cement holes **348,** as shown in the figures.

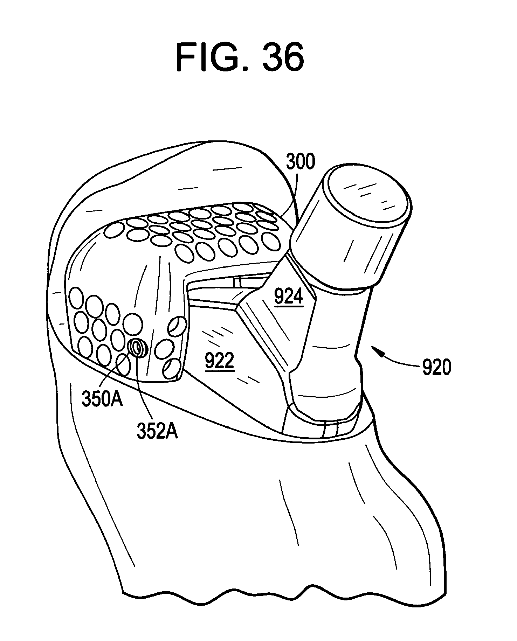

In one embodiment, the device **300** may be further held in place using one or more set screws. **FIG. 36-37** are perspective and side views, respectively, of the drug delivery device **300**, illustrating set screws maintaining a position of the device **300** against a hip stem **922** of the implanted hip prosthesis **920.** As shown, the device **300** may include a forward set screw hole **350A** that extends through the front pillar **304** from the front face to the rear face, and a rear set screw hole **350B** that extends through the rear pillar **306** from the rear face to the front face. Set screws **352A, 352B** may be positioned in the corresponding set screw holes **350A, 350B** to maintain a position of the device **300** against a hip stem **922** of the implanted hip prosthesis **920**. In such embodiments, the device **300** may be reconfigured to accommodate the set screw holes **350,** such as by removing one or more of the reservoirs **342** or cement holes **348**, although such reconfiguration may not be necessary. It should be noted that set screws may also be used with the device 200, in which case the device **200** may have corresponding set screw holes. Further, any combination of these or other fixation means may be used to secure the device to the surgically prepared bone section and/or the implant, as described above.

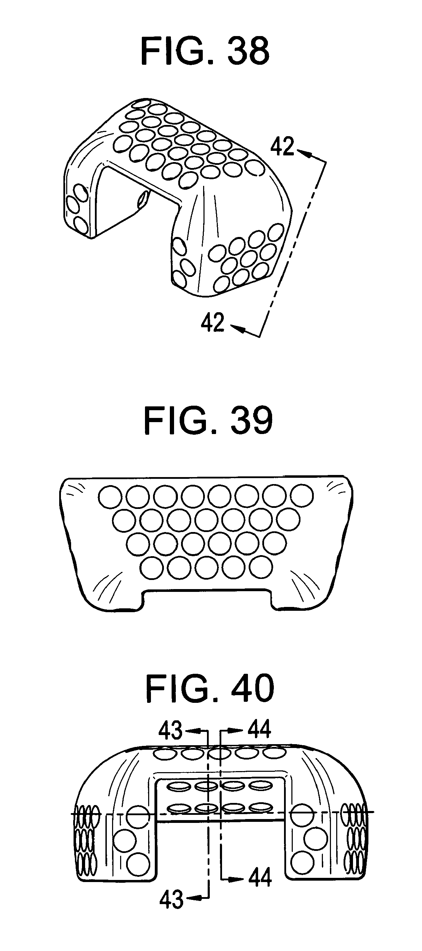

**FIGS. 38-48** show various views of the drug delivery device **300**, illustrating example dimensions of the device. To accommodate hip prostheses **920** of various sizes, the body **302** may be made relatively smaller or larger, such as by varying one or more dimensions of the device **300**, although the illustrated embodiment may be suited for use with a number of hip prostheses **920** of conventional sizes. It should be noted that devices **300** of different sizes may have different locations, sizes, and numbers of reservoirs to accommodate the desired volume of drug formulation while maintaining the mechanical integrity of the device.

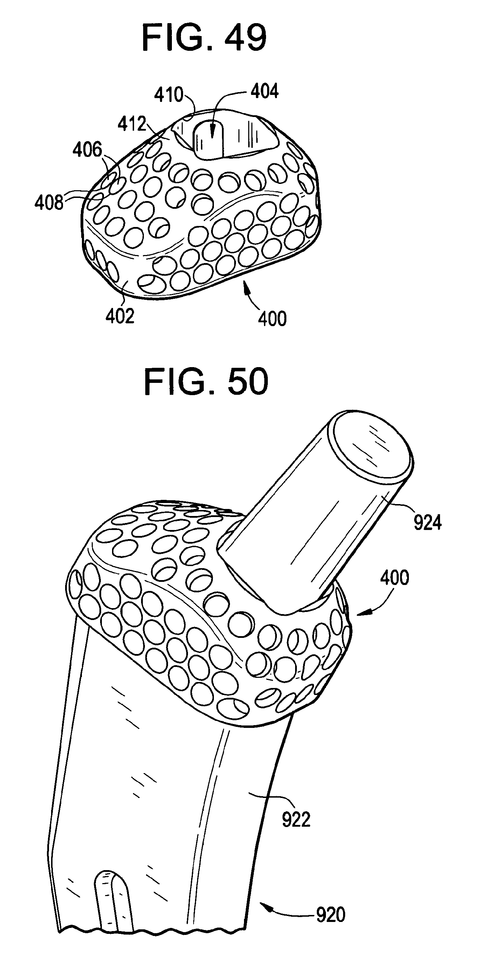

**FIGS. 49-55** illustrate another embodiment of a drug delivery device **400** adapted for use with a standard hip replacement prosthesis, such as the prosthesis **920** described above. As shown in **FIG. 49****,** which is a perspective view of the drug delivery device **400,** the device **400** may generally include a body **402** formed in the shape of a closed sleeve or cap. As shown in **FIGS. 50-55**, which illustrate the drug delivery device 400 implanted about a hip prosthesis **920,** the cap shape of the body **402** may permit fitting the body **402** over a portion of the implanted prosthesis **920,** such as the area formerly occupied by the femoral head. The body **402** may also include a hip stem aperture **404** that may be sized and shaped to receive a portion of the neck **924** of the hip stem **922**. The shape of the body **402** may permit installing the device **400** at a height that is even with or lower than existing stem flats on the hip stem **922**, for reasons described below. As shown, the hip stem **922** may be an unmodified, tapered hip stem.

The body **402** may include an inner surface **410 t**hat defines the aperture **404**.

Thus, the inner surface **410** may be sized and shaped for placement in confronting relation to the neck **924** of the hip stem **922.** The body **402** may also include an outer surface **412** that is outwardly directed toward the adjacent anatomy once he device **400** is implanted.

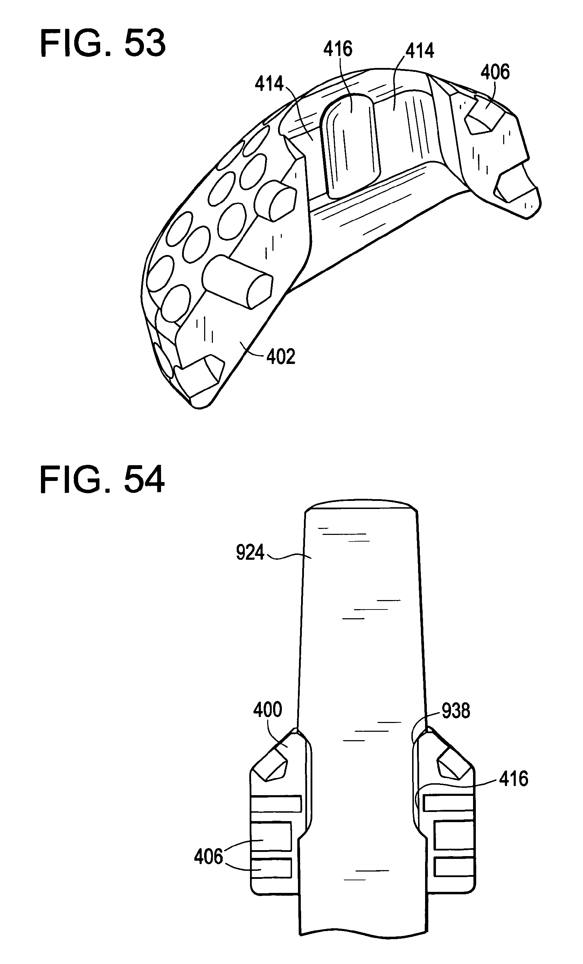

A number of reservoirs **406** may be formed in the body **402** for containing a drug formulation. The reservoirs **406** may be in communication with openings **408** formed in the outer surface **412,** so that the drug formulation may be released from the reservoirs **406** through the openings **408** and into the implantation site. The reservoirs **406** may have varying cross-sectional shapes and sizes depending on the position on the body **402**, as a varying amount of space may be available for accommodating the reservoirs **406** within the body **402**. For example, each reservoir **406** may have a diameter of about 3 mm and a depth varying from about 2.8 mm to about 5 mm, depending on the location on the body **402.** The reservoirs **406** of varying sizes and shapes can be seen in **FIG. 53****,** which is a perspective cross-sectional view of the device **400**. In total, the reservoirs **406** may be sized to store a total volume of about 2 mL of the drug formulation, which may be an antibiotic.

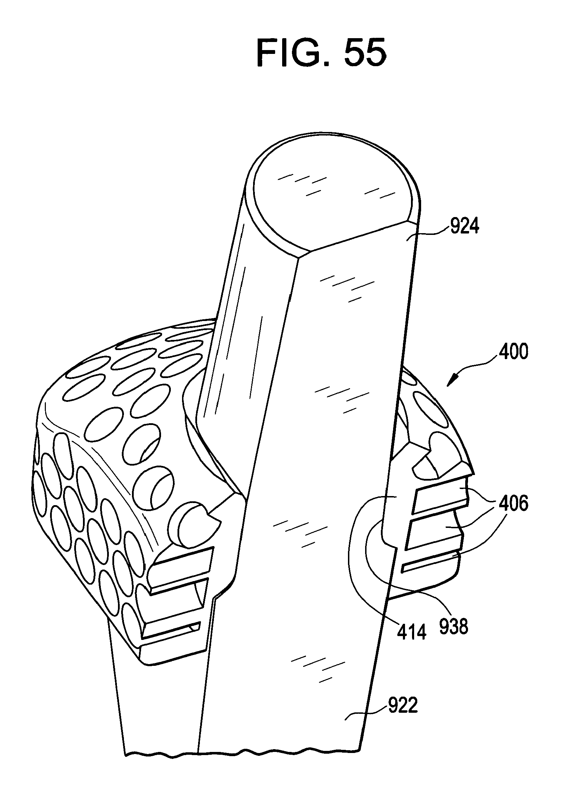

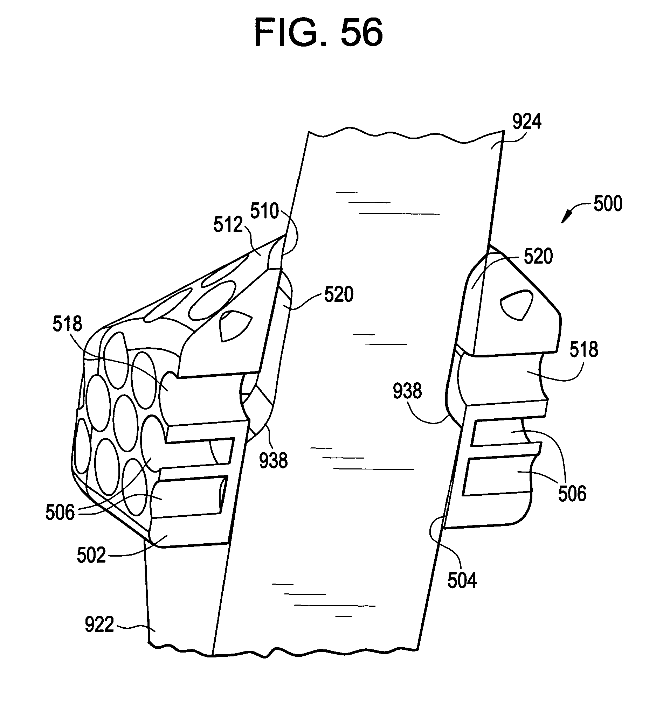

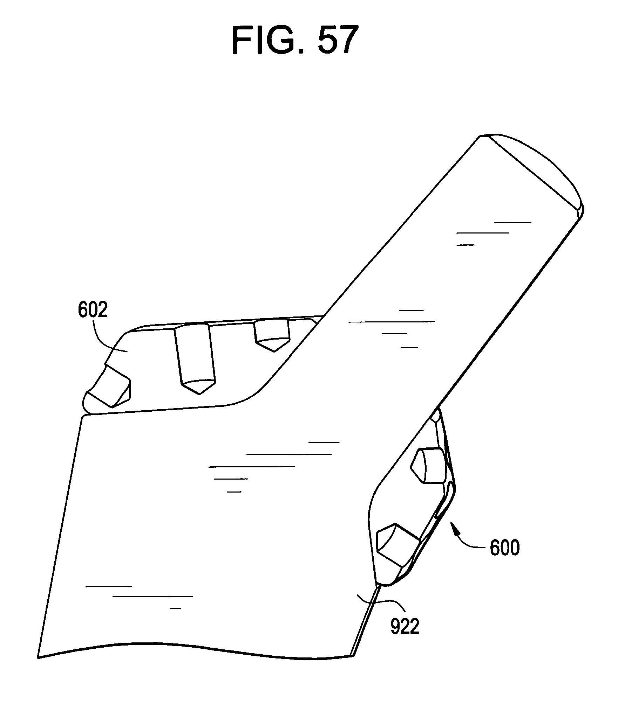

As shown in **FIG. 53**, the device **400** may include one or more protruding flat areas **414** and one or more relief areas **416**. The protruding flat areas **414** and the relief areas **416** may be positioned in the hip stem aperture **404**. The protruding flat areas **414** may be adapted to engage stem flats **938** on the neck **924** of the hip stem **922** to secure the device **400** to the hip stem **922**, and the relief areas **416** may permit the device **400** to deflect so that the device **400** may be slid onto the neck **924** of the hip stem **922** despite the protruding flat areas **414.** For example, **FIG. 54** is a front cross-sectional view of the device **400** implanted about the hip stem **922,** taken through the relief areas **416** positioned adjacent to the stem flats **938**, and **FIG. 55** is a perspective cross-sectional view of the device **400** implanted about the hip stem **922,** taken through the protruding flat areas **414** positioned adjacent to the stem flats **938.** However, the device **400** may be secured to the hip stem **922** in other manners. For example, the device **400** may be adapted to be secured to the hip stem **922** using cement, such as a bone cement. Such an embodiment is shown in **FIG. 56**, which is a perspective cross-sectional view of an embodiment of the device **500,** illustrating cement ports **518** and cement receiving areas **520** of the device **500**. The cement receiving areas **520** are designed to receive a volume of cement, such as bone cement. The cement ports **518** may extend through the body **502** from the outer surface **512** to the inner surface **510** within the aperture **504**. The cement ports **518** may be in communication with the cement receiving areas **520**, which are sized and shaped to receive a volume of cement. When the device **500** is positioned about the neck **924** of the hip stem **922**, the cement receiving areas **520** are spaced apart from the neck **924**, so that cement injected through the cement ports **518** can become positioning in the cement receiving areas **520** to secure the device 500 against the hip stem **922**, such as about the stem flats **938** in the area of the neck **924**. In other embodiments, the device may be molded to mate with the hip stem. For example, **FIG. 57** is a cross-sectional view of another embodiment of a drug delivery device **600,** illustrating the device implanted about a tapered hip stem **922.** As shown, the device **600** may be molded such that an inner profile of the device **600** matches an outer profile of the tapered hip stem **922.** In embodiments, the inner surface of the device **600** circumscribing the aperture may include a plastic material that is relatively soft in comparison to the rest of the body. The plastic material may form an inner liner that deforms, such as when the aperture receives the hip stem **922,** to assist in securing the device **600** about the hip stem **922.**

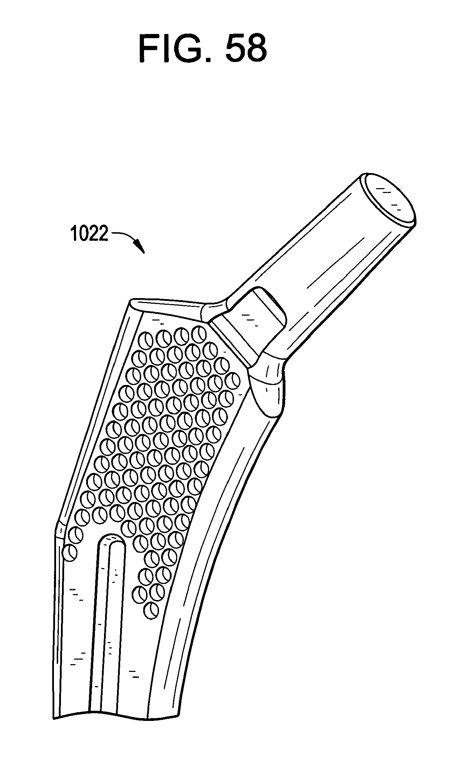

**FIG. 58** is a cross-sectional view of an embodiment of a tapered hip stem **1022**, which includes multiple discrete reservoirs for holding a drug formulation. The tapered hip stem **1022** can be used alone or in combination with any of the devices described above.

Various embodiments of the drug delivery device described above may be useful in drug therapy. In one case, the drug delivery devices may be used in at least part of an antibiotic treatment for a orthopedic surgery patient, in either prophylaxis or treatment of an infection. In one embodiment, the treatment provided by the drug delivery device may be over a relatively short term, such as a term of up to three months, in association with a hip replacement procedure, such as a cement-less total hip arthroplasty (THA) procedure or other THA procedures. Such short term prophylactic antibiotic therapy may reduce the incidence of post-operative infection associated with the implantation procedure.

Each of the devices described above may be formed of, for example, a biocompatible metal, , a ceramic, a degradable or non-degradable polymer, or combinations thereof. In some embodiments, the body may be formed from a core covered in a drug-eluting coating. The coating may be a blend of a polymer and a drug, such that the drug is released from the polymer, for example by diffusion and/or surface dissolution/erosion when the polymer is bioerodible or biodegradable. In embodiments in which the body has a core, the reservoirs may be omitted. Alternatively, the body may have the reservoirs and the drug-eluting coating.

The device may be designed from permanent materials, such as metals or ceramics, or resorbable materials. In embodiments in which the device is formed from resorbable materials, the device may release the drug formulation of a period of time that is relatively shorter than the period of time over which the device resorbs. For example, the device may deliver all or substantially all of the drug formulation over a period of about three months, and the device may resorb over a period of about one year.

### Making the Devices

The basic methods of fabricating and assembling the elongated body portion described herein are known or can be readily adapted from techniques known in the art. Reservoirs may be created in the device body simultaneously with formation of the device body, or they may be formed in the device body after the device body is made.

Representative fabrication techniques include conventional material forming processes, such as those known in the art for forming contoured metal, ceramic, or polymeric articles. In some embodiments, the devices may be made using methods that include MEMS fabrication processes, microfabrication processes, or other micromachining processes, various drilling techniques (e.g., laser, mechanical, and ultrasonic drilling), electrical discharge machining (EDM), and build-up or lamination techniques, such as LTCC (low temperature co-fired ceramics). Microfabrication methods include lithography and etching, injection molding and hot embossing, electroforming/electroplating, microdrilling (e.g., laser drilling), micromilling, electrical discharge machining (EDM), photopolymerization, surface micromachining, high-aspect ratio methods (e.g., LIGA), micro stereo lithography, silicon micromachining, rapid prototyping, and DEEMO (Dry Etching, Electroplating, Molding). Reservoirs may be fabricated into metal body portions by techniques known in the art, including laser etching, laser jet etching, micro-EDM, oxide film laser lithography, and computerized numerical control machining.

The surface of the reservoir optionally may be treated or coated to alter one or more properties of the surface. Examples of such properties include hydrophilicity/hydrophobicity, wetting properties (surface energies, contact angles, etc.), surface roughness, electrical charge, release characteristics, and the like.

U.S. Patent No. 6,808,522; U.S. Patent No. 6,123,861; U.S. Patent No. 6,527,762; and U.S. Patent No. 6,976,982 describe micromolding and other techniques for making certain reservoir device bodies and caps. These methods may be modified and adapted, based on the teachings herein, to make the implant devices described herein.

### Implanting/Using the Devices

The drug delivery device typically is implanted during the surgical procedure for implanting the prosthetic device. The surgical procedure may be a least invasive procedure that is performed, for example, through a 2 to 3 inch incision. The procedure typically involves preparing the bone tissue, which may include removing or resurfacing part of the bone and other tissues. The prosthetic device is then implanted in the surgically prepared bone in a conventional manner.

In one embodiment, the drug delivery device may be secured to the prosthetic device before the prosthetic device is implanted at the tissue site. In another embodiment, the drug delivery device may be secured to the prosthetic device after the prosthetic device is implanted at the tissue site. The device may be implanted into a patient (such as a human or other vertebrate animal) using standard surgical implantation techniques.

Robotic guidance systems or imaging techniques may be employed to confirm proper positioning of the implant.

Following implantation into the patient, the devices releases one or more drugs locally at the tissue site of implantation at a preselected delivery profile (e.g., dosing schedule) based on the design of the particular device as prescribed by the patient's physician. In one embodiment, the devices store and release an effective amount of at least one drug formulation over an extended period, e.g., between about 1 month and about 3 months.

Release of the drug(s) may be passively controlled as described hereinabove.

Modifications and variations of the methods and devices described herein will be obvious to those skilled in the art from the foregoing detailed description. Such modifications and variations are intended to come within the scope of the appended claims.

## Claims

1. An implantable drug delivery device adapted to be installed at or adjacent an implantable orthopedic prosthetic device inserted onto a surgically prepared bone section of a human or other animal, comprising:
a body having a core formed from a non-biodegradable material formed in the shape of a sleeve and having an inner surface and an outwardly directed outer surface; and
wherein said outer surface has a drug formulation for release thereof into the body of the animal following implantation of the prosthetic device within the body of the animal.

2. The drug delivery device of claim 1, wherein the body defines an open shape having free ends.

3. The drug delivery device of claim 1, wherein the body defines a closed annular shape.

4. The drug delivery device of claim 1, wherein said prosthetic device is a hip replacement joint having a femoral portion including a hip stem having a neck;
wherein a portion of the body is adapted to be cemented to at least a portion of one or both of the following: said hip stem and said surgically prepared bone section; and
wherein said body in the shape of a sleeve defines an aperture adapted to receive a portion of said hip stem in confronting relation around three sides of said hip stem.

5. The drug delivery device of claim 1, wherein said prosthetic device is a hip replacement joint having a femoral portion including a hip stem having a neck;
wherein the body is adapted to receive said hip stem with said inner surface of the body in surrounding relation to said neck.

6. The drug delivery device of claim 2, wherein said prosthetic device is a knee replacement joint having an articular surface;
wherein said body is adapted to engage a peripheral portion of said articular surface; and
wherein said sleeve has an approximate C-shaped extent, portions of said sleeve being smooth in regions to be overlaid by muscle or tendon tissue.

7. An implantable drug delivery device adapted to be installed at or adjacent an implantable orthopedic prosthetic device inserted onto a surgically prepared bone section of a human or other animal, comprising:
a body having a radially inwardly directed inner surface defining an aperture, and a radially outwardly directed outer surface, and said body defining an open shape having two free ends;
said body having a thickness dimension defined in a generally radial direction extending from said inner surface to said outer surface;
wherein said aperture extends over the length of the thickness dimension; and
wherein said outer surface holds a drug formulation for release thereof into the body of the animal following implantation of the prosthetic device within the body of the animal.

8. The drug delivery device of any one of claims 1 to 7, wherein the outer surface defines at least one pocket for holding said drug formulation.

9. The drug delivery device of claim 7, wherein said prosthetic device is a hip replacement joint having a femoral portion including a hip stem having a neck;
wherein said body is adapted to be cemented to at least a portion of said hip stem or said surgically prepared bone section; and
wherein said aperture is adapted to receive a portion of said hip stem in confronting relation around three sides of said hip stem.

10. An implantable drug delivery device adapted to be installed on an implantable orthopedic prosthetic device inserted onto a surgically prepared bone section of a human or other animal, comprising:
a body having a first wall and an outer surface defining at least one pocket for holding a drug formulation, said first wall adapted to be cemented to at least one of a portion of said implantable prosthetic device or said surgically prepared bone section;
an aperture formed in said body and extending through said body, said aperture adapted to receive a portion of said implantable prosthetic device; and
said at least one pocket holds a drug formulation for release thereof into the body of the animal following implantation of the prosthetic device within the body of the animal.

11. The drug delivery device of claim 10, wherein said prosthetic device is a hip replacement joint having a femoral portion including a hip stem having a neck;
wherein said first wall is adapted to be cemented to at least a portion of said hip stem or said surgically prepared bone section; and
wherein said aperture is adapted to receive a portion of said hip stem in confronting relation around three sides of said hip stem.

12. The drug delivery device of any one of claims 1 to 11, wherein said drug formulation comprises an antibiotic.

13. The drug delivery device of any one of claims 1 to 12, wherein the body further comprises a fixation means chosen from the group of fixation means consisting of a hole adapted to receive a screw, a pin, a suture, a cement, or a glue; a detent adapted to engage a groove; an indentation adapted to receive a cement or glue; a snap fitting; a pressure fitting; a screw fitting; a screw; a pin; a suture; a cement; a glue; and a groove.

14. The drug delivery device of any one of claims 1 to 13, wherein the body is formed of a material belonging to the group of materials consisting of: metal, metal alloy, ceramic, and polymers.

15. The drug delivery device of claim 1, 7, or 10, wherein said prosthetic device is a portion of a hip or knee replacement joint.

16. A system for use in the delivery of a drug to a human or other animal having an implantable orthopedic prosthetic device, comprising:
an orthopedic prosthetic device suitable for implantation on a surgically prepared bone section of the human or other animal;
an implantable drug delivery device having an aperture; which surrounds a portion of the implanted prosthetic device with the aperture of the implantable drug delivery device in a manner preventing relative motion between the prosthetic device and the drug delivery device.
